(19) Europäisches Patentamt
European Patent Office
Office européen des brevets

(11) Veröffentlichungsnummer: **0 138 149 B1**

(12) # EUROPÄISCHE PATENTSCHRIFT

(45) Veröffentlichungstag der Patentschrift: **18.01.89**

(51) Int. Cl.⁴: **C 07 D 231/40**, C 07 D 231/38, C 07 C 121/45, A 01 N 43/56

(21) Anmeldenummer: **84111782.3**

(22) Anmeldetag: **03.10.84**

(54) **Substituierte 5-Acylamino-1-phenylpyrazole.**

(30) Priorität: **15.10.83 DE 3337543**
**06.06.84 DE 3420985**

(43) Veröffentlichungstag der Anmeldung: **24.04.85 Patentblatt 85/17**

(45) Bekanntmachung des Hinweises auf die Patenterteilung: **18.01.89 Patentblatt 89/3**

(84) Benannte Vertragsstaaten: **AT BE CH DE FR GB IT LI NL SE**

(56) Entgegenhaltungen:
**EP-A- 0 034 945**
**DE-A- 3 045 903**
**DE-A- 3 226 513**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Gehring, Reinhold, Dr., Dasnöckel 49, D-5600 Wuppertal 11 (DE)**
Erfinder: **Klauke, Erich, Dr., Eichendorffweg 8, D-5068 Odenthal (DE)**
Erfinder: **Schallner, Otto, Dr., Noldeweg 22, D-4019 Monheim (DE)**
Erfinder: **Stetter, Jörg, Dr., Gellertweg 4, D-5600 Wuppertal 1 (DE)**
Erfinder: **Santel, Hans-Joachim, Dr., Gerstenkamp 19, D-5000 Köln 80 (DE)**
Erfinder: **Schmidt, Robert R. Dr., Im Waldwinkel 110, D-5060 Bergisch-Gladbach 2 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft neue substituierte 5-Acylamino-1-phenylpyrazole, ein Verfahren zu ihrer Herstellung und ihre Verwendung als Herbizide.

Es ist bereits bekannt, dass bestimmte substituierte 5-Acylamino-1-phenylpyrazole, wie beispielsweise das 4-Cyano-5-propionamido-1-(2,4,6-trichlorphenyl)-pyrazol, herbizide Eigenschaften besitzen (vergleiche z.B. DE-OS 3 226 513).

Die hebizide Wirkung dieser bekannten Verbindungen gegenüber Unkräutern, ebenso wie deren Verträglichkeit gegenüber wichtigen Kulturpflanzen ist jedoch nicht immer in allen Fällen voll befriedigend.

Es wurden neue substituierte 5-Acylamino-1-phenylpyrazole der allgemeinen Formel (I),

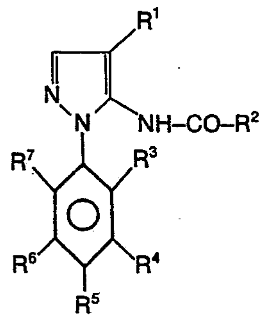

(I)

in welcher

$R^1$ für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl oder Alkinylaminocarbonyl mit jeweils bis zu 4 Kohlenstoffatomen in den einzelnen Alkylteilen steht,

$R^2$ für Wasserstoff, für jeweils geradkettiges oder verzweigtes Alkyl, Alkenyl oder Alkinyl, mit bis zu 6 Kohlenstoffatomen, für Cycloalkyl mit 3 bis 7 Kohlenstoffatomen, für jeweils geradkettiges und verzweigtes Alkoxyalkyl oder Alkylthioalkyl mit jeweils bis zu 4 Kohlenstoffatomen in den einzelnen Alkylteilen oder für geradkettiges oder verzweigtes Halogenalkyl mit bis zu 6 Kohlenstoffatomen und bis zu 9 gleichen oder verschiedenen Halogenatomen, oder für einfach oder mehrfach, gleich oder verschieden substituiertes Phenyl steht, wobei als Substituenten infrage kommen: Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen, und

$R^3$, $R^4$, $R^5$, $R^6$ und $R^7$ unabhängig voneinander für Wasserstoff, Fluor, Chlor, Brom, Iod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X)_n-R^8$ stehen, wobei

X für Sauerstoff, Schwefel, Sulfinyl oder Sulfonyl steht,

n für 0 oder 1 steht und $R^8$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$, $R^5$, $R^6$ oder $R^7$ für einen Rest $-(X)_n-R^8$ steht, wobei jedoch nicht gleichzeitig $R^1$ für Cyano und $R^5$ für Trifluormethyl stehen,

gefunden.

Weiterhin wurde gefunden, dass man die neuen substituierten 5-Acylamino-1-phenylpyrazole der allgemeinen Formel (I) erhält, wenn man 5-Aminopyrazole der Formel (II)

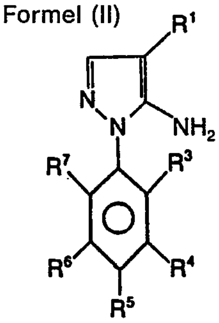

(II)

in welcher

$R^1$, $R^3$, $R^4$, $R^5$, $R^6$ und $R^7$ die oben angegebene Bedeutung haben,

mit Acylierungsmitteln der Formel (III)

$$R^2-CO-A \quad (III)$$

in welcher

$R^2$ die oben angegebene Bedeutung hat und

A für eine aktivierende Abgangsgruppe steht,

gegebenenfalls in Gegenwart eines Verdünnungsmittels und gegebenenfalls in Gegenwart eines Säurebindemittels umsetzt.

Schliesslich wurde gefunden, dass die neuen substituierten 5-Acylamino-1-phenylpyrazole der Formel (I) herbizide Eigenschaften, insbesondere auch selektiv-herbizide Eigenschaften besitzen.

Überraschenderweise besitzen die neuen substituierten 5-Acylamino-1-phenylpyrazole der Formel (I) eine bessere herbizide Wirksamkeit gegenüber Schadpflanzen, bei gleichzeitiger besserer Verträglichkeit gegenüber wichtigen Nutzpflanzen, als beispielsweise das aus dem Stand der Technik bekannte 4-Cyano-5-propionamido-1-(2,4,6-trichlorphenyl)-pyrazol, welches eine chemisch und wirkungsmässig naheliegende Verbindung ist.

Die neuen substituierten 5-Acylamino-1-phenylpyrazole sind durch die Formel (I) allgemein definiert. Bevorzugt sind Verbindungen der Formel (I), bei welchen

$R^1$ für Cyano, Methoxycarbonyl, Ethoxycarbonyl, Allyloxycarbonyl, Propargyloxycarbonyl, Aminocarbonyl, Methylaminocarbonyl, Ethylaminocarbonyl steht,

$R^2$ für Wasserstoff, Methyl-, Ethyl-, n- und i-Propyl, n-, i-, s- und t-Butyl, Cyclopropyl, Methylthiomethyl, Cyclopentyl, Cyclohexyl, Ethoxymethyl, Methoxymethyl, Methoxyethyl, Ethoxyethyl, Chlormethyl, Dichlormethyl, Trichlormethyl, Trifluormethyl, Dichlorfluormethyl, Difluorchlormethyl oder Pentafluorethyl, oder für ein- bis dreifach, gleich oder verschieden durch Fluor, Chlor, Brom, $NO_2$, Methyl oder Methoxy substituiertes Phenyl steht und

$R^3$, $R^4$, $R^5$, $R^6$ und $R^7$ unabhängig voneinander für Wasserstoff, Fluor, Chlor, Brom, Cyano, Nitro, Methyl, Ethyl, n- und i-Propyl, Methoxy, Ethoxy, Methylsulfonyl, Methoxycarbonyl, Ethoxycarbonyl oder für einen Rest $-(X)_n-R^8$ stehen, wobei X für Sauerstoff, Schwefel, Sulfinyl oder Sulfonyl steht, n für 0 oder 1 steht und $R^8$ für Trifluormethyl, Trichlormethyl, Dichlorfluormethyl, Difluorchlormethyl, Dichlormethyl, Chlormethyl, Difluormethyl, Pentafluorethyl, Tetrafluorethyl, Trifluorchlorethyl, Trifluorethyl, Difluordichlorethyl, Trifluordichlorethyl oder Pentachlorethyl stehen, mit der Massgabe, dass mindestens einer der Reste $R^3$, $R^4$, $R^5$, $R^6$ oder $R^7$ für einen Rest $-(X)_n-R^8$ steht, wobei jedoch nicht gleichzeitig $R^1$ für Cyano und $R^5$ für Trifluormethyl stehen.

Im einzelnen seien ausser den bei den Herstellungsbeispielen genannten Verbindungen, die folgenden Verbindungen der allgemeinen Formel (I) genannt:

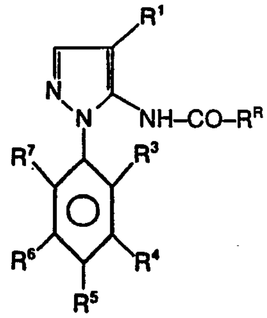

(I)

Tabelle 1

| $R^1$ | $R^2$ | $R^3$ | $R^4$ | $R^5$ | $R^6$ | $R^7$ |
|---|---|---|---|---|---|---|
| CN | H | F | H | $OCF_3$ | H | H |
| CN | H | F | H | $OCF_3$ | H | F |
| CN | H | F | F | $OCF_3$ | F | F |
| CN | H | Cl | H | $OCF_3$ | H | H |
| CN | H | Cl | H | $OCF_3$ | H | Cl |
| CN | H | Cl | Cl | $OCF_3$ | H | H |
| CN | H | Cl | H | $OCF_3$ | H | F |
| CN | H | Br | H | $OCF_3$ | H | H |
| CN | H | Br | H | $OCF_3$ | H | Br |
| CN | $CH_3$ | F | H | $OCF_3$ | H | H |
| CN | $CH_3$ | F | H | $OCF_3$ | H | F |
| CN | $CH_3$ | F | F | $OCF_3$ | F | F |
| CN | $CH_3$ | Cl | H | $OCF_3$ | H | H |
| CN | $CH_3$ | Cl | H | $OCF_3$ | H | Cl |
| CN | $CH_3$ | Cl | Cl | $OCF_3$ | H | H |
| CN | $CH_3$ | Cl | H | $OCF_3$ | H | F |
| CN | $CH_3$ | Br | H | $OCF_3$ | H | H |
| CN | $CH_3$ | Br | H | $OCF_3$ | H | Br |
| CN | $C_2H_5$ | F | H | $OCF_3$ | H | H |
| CN | $C_2H_5$ | F | H | $OCF_3$ | H | F |
| CN | $C_2H_5$ | F | F | $OCF_3$ | F | F |
| CN | $C_2H_5$ | Cl | H | $OCF_3$ | H | H |
| CN | $C_2H_5$ | Cl | H | $OCF_3$ | H | Cl |
| CN | $C_2H_5$ | Cl | Cl | $OCF_3$ | H | H |
| CN | $C_2H_5$ | Cl | H | $OCF_3$ | H | F |
| CN | $C_2H_5$ | Br | H | $OCF_3$ | H | H |
| CN | $C_2H_5$ | Br | H | $OCF_3$ | H | Br |
| CN | 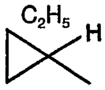 | F | H | $OCF_3$ | H | H |
| CN | 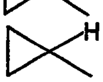 | F | H | $OCF_3$ | H | F |
| CN | 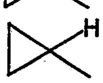 | F | F | $OCF_3$ | F | F |
| CN | 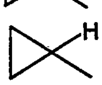 | Cl | H | $OCF_3$ | H | H |
| CN | 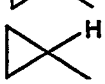 | Cl | H | $-OCF_3$ | H | Cl |
| CN | 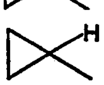 | Cl | Cl | $-OCF_3$ | H | H |
| CN | 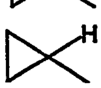 | Cl | H | $-OCF_3$ | H | F |
| CN | 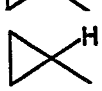 | Br | H | $-OCF_3$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | 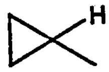 | Br | H | $-OCF_3$ | H | Br |
| CN | $CH_3$ | Cl | H | $-S-CH_2CF_3$ | H | H |
| CN | $CH_3$ | Cl | H | $-S-CH_2CF_3$ | H | Cl |
| CN | $CH_3$ | Br | H | $-S-CH_2CF_3$ | H | H |
| CN | $CH_3$ | Br | H | $-S-CH_2CF_3$ | H | Br |
| CN | $CH_3$ | Cl | Cl | $-S-CH_2CF_3$ | H | H |
| CN | $C_2H_5$ | Cl | H | $-S-CH_2CF_3$ | H | H |
| CN | $C_2H_5$ | Cl | H | $-S-CH_2CF_3$ | H | Cl |
| CN | $C_2H_5$ | Br | H | $-S-CH_2CF_3$ | H | H |
| CN | $C_2H_5$ | Br | H | $-S-CH_2CF_3$ | H | Br |
| CN | $C_2H_5$ | Cl | Cl | $-S-CH_2CF_3$ | H | H |
| CN | 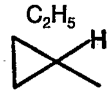 | Cl | H | $-S-CH_2CF_3$ | H | H |
| CN | 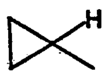 | CL | H | $-S-CH_2CF_3$ | H | Cl |
| CN | 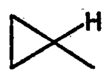 | Br | H | $-S-CH_2CF_3$ | H | H |
| CN | 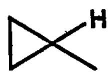 | Br | H | $-S-CH_2CF_3$ | H | Br |
| CN | 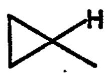 | Cl | Cl | $-S-CH_2CF_3$ | H | H |
| CN | $CH_3OCH_2-$ | Cl | H | $-S-CH_2CF_3$ | H | H |
| CN | $CH_3OCH_2-$ | Cl | H | $-S-CH_2CF_3$ | H | Cl |
| CN | $CH_3OCH_2-$ | Br | H | $-S-CH_2CF_3$ | H | H |
| CN | $CH_3OCH_2-$ | Br | H | $-S-CH_2CF_3$ | H | Br |
| CN | $CH_3OCH_2-$ | Cl | Cl | $-S-CH_2CF_3$ | H | H |
| CN | $CH_3$ | Cl | H | $-O-CH_2CF_3$ | H | H |
| CN | $CH_3$ | Cl | H | $-O-CH_2CF_3$ | H | Cl |
| CN | $CH_3$ | Br | H | $-O-CH_2CF_3$ | H | H |
| CN | $CH_3$ | Br | H | $-O-CH_2CF_3$ | H | Br |
| CN | $C_2H_5$ | Cl | H | $-O-CH_2CF_3$ | H | H |
| CN | $C_2H_5$ | Cl | H | $-O-CH_2CF_3$ | H | Cl |
| CN | $C_2H_5$ | Br | H | $-O-CH_2CF_3$ | H | H |
| CN | $C_2H_5$ | Br | H | $-O-CH_2CF_3$ | H | Br |
| CN | 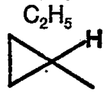 | Cl | H | $-O-CH_2CF_3$ | H | H |
| CN | 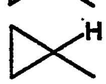 | Cl | H | $-O-CH_2CF_3$ | H | Cl |
| CN | 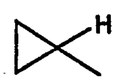 | Br | H | $-O-CH_2CF_3$ | H | H |
| CN | 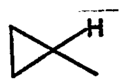 | Br | H | $-O-CH_2CF_3$ | H | Br |
| CN | $CH_3OCH_2-$ | Cl | H | $-O-CH_2CF_3$ | H | H |
| CN | $CH_3OCH_2-$ | Cl | H | $-O-CH_2CF_3$ | H | Cl |
| CN | $CH_3OCH_2-$ | Br | H | $-O-CH_2CF_3$ | H | H |
| CN | $CH_3OCH_2-$ | Br | H | $-O-CH_2CF_3$ | H | Br |
| CN | $CH_3$ | $SCF_3$ | H | Cl | H | H |
| CN | $C_2H_5$ | $SCF_3$ | H | Cl | H | H |
| CN | $CH_3$ | $SCF_3$ | H | Cl | H | Cl |
| CN | $C_2H_5$ | $SCF_3$ | H | Cl | H | Cl |
| CN | H | F | H | $SCF_3$ | H | H |
| CN | H | F | H | $SCF_3$ | H | F |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | H | F | F | $SCF_3$ | F | F |
| CN | H | Cl | H | $SCF_3$ | H | H |
| CN | H | Cl | H | $SCF_3$ | H | Cl |
| CN | H | Cl | Cl | $SCF_3$ | H | H |
| CN | H | Cl | H | $SCF_3$ | H | F |
| CN | H | Br | H | $SCF_3$ | H | H |
| CN | H | Br | H | $SCF_3$ | H | Br |
| CN | $CH_3$ | F | H | $SCF_3$ | H | H |
| CN | $CH_3$ | F | H | $SCF_3$ | H | F |
| CN | $CH_3$ | F | F | $SCF_3$ | F | F |
| CN | $CH_3$ | Cl | H | $SCF_3$ | H | H |
| CN | $CH_3$ | Cl | H | $SCF_3$ | H | Cl |
| CN | $CH_3$ | Cl | Cl | $SCF_3$ | H | H |
| CN | $CH_3$ | Cl | H | $SCF_3$ | H | F |
| CN | $CH_3$ | Br | H | $SCF_3$ | H | H |
| CN | $CH_3$ | Br | H | $SCF_3$ | H | Br |
| CN | $C_2H_5$ | F | H | $SCF_3$ | H | H |
| CN | $C_2H_5$ | F | H | $SCF_3$ | H | F |
| CN | $C_2H_5$ | F | H | $SCF_3$ | F | F |
| CN | $C_2H_5$ | Cl | H | $SCF_3$ | H | H |
| CN | $C_2H_5$ | Cl | H | $SCF_3$ | H | Cl |
| CN | $C_2H_5$ | Cl | H | $SCF_3$ | H | F |
| CN | $C_2H_5$ | Br | H | $SCF_3$ | H | H |
| CN | 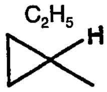 | F | H | $SCF_3$ | H | H |
| CN | 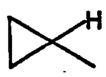 | F | H | $SCF_3$ | H | F |
| CN | 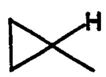 | F | F | $SCF_3$ | F | F |
| CN | 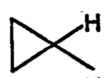 | Cl | H | $SCF_3$ | H | H |
| CN | 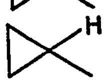 | Cl | H | $SCF_3$ | H | Cl |
| CN | 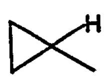 | Cl | Cl | $SCF_3$ | H | H |
| CN | 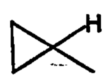 | Cl | H | $SCF_3$ | H | F |
| CN | 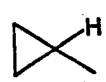 | Br | H | $SCF_3$ | H | H |
| CN | 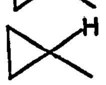 | Br | H | $SCF_3$ | H | Br |
| CN | H | $CF_3$ | H | $-SO_2CH_3$ | H | H |
| CN | H | $CF_3$ | H | $-SO_2CH_3$ | H | H |
| CN | H | $CF_3$ | H | $-SCF_3$ | H | H |
| CN | H | $OCF_3$ | H | $-CF_3$ | H | H |
| CN | H | $OCF_3$ | H | $-OCF_3$ | H | H |
| CN | $CH_3$ | $CF_3$ | H | $-SO_2CH_3$ | H | H |
| CN | $CH_3$ | $CF_3$ | H | $-SO_2CH_3$ | H | H |
| CN | $CH_3$ | $CF_3$ | H | $-SCF_3$ | H | H |
| CN | $CH_3$ | $OCF_3$ | H | $-OCF_3$ | H | H |
| CN | $CH_3$ | $OCF_3$ | H | $-CF_3$ | H | H |
| CN | $C_2H_5$ | $CF_3$ | H | $-SO_2CH_3$ | H | H |
| CN | $C_2H_5$ | $CF_3$ | H | $-SO_2CH_3$ | H | H |
| CN | $C_2H_5$ | $CF_3$ | H | $-SCF_3$ | H | H |
| CN | $C_2H_5$ | $OCF_3$ | H | $-OCF_3$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | $C_2H_5$ | $OCF_3$ | H | $-CF_3$ | H | H |
| CN | 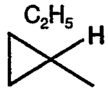 | $CF_3$ | H | $-SO_2CH_3$ | H | H |
| CN | 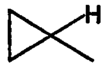 | $CF_3$ | H | $-SO_2CH_3$ | H | H |
| CN | 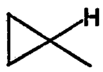 | $CF_3$ | H | $-SCF_3$ | H | H |
| CN | 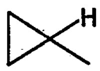 | $OCF_3$ | H | $-OCF_3$ | H | H |
| CN | 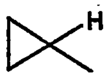 | $OCF_3$ | H | $-CF_3$ | H | H |
| CN | H | Cl | H | $-SCHF_2$ | H | H |
| CN | H | Cl | H | $-SCHF_2$ | H | Cl |
| CN | H | Br | H | $-SCHF_2$ | H | H |
| CN | H | Br | H | $-SCHF_2$ | H | Br |
| CN | $CH_3$ | Cl | H | $-SCHF_2$ | H | H |
| CN | $CH_3$ | Cl | H | $-SCHF_2$ | H | Cl |
| CN | $CH_3$ | Br | H | $-SCHF_2$ | H | H |
| CN | $CH_3$ | Br | H | $-SCHF_2$ | H | Br |
| CN | $C_2H_5$ | Cl | H | $-SCHF_2$ | H | H |
| CN | $C_2H_5$ | Cl | H | $-SCHF_2$ | H | Cl |
| CN | $C_2H_5$ | Br | H | $-SCHF_2$ | H | H |
| CN | $C_2H_5$ | Br | H | $-SCHF_2$ | H | Br |
| CN | 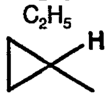 | Cl | H | $-SCHF_2$ | H | H |
| CN | 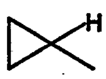 | Cl | H | $-SCHF_2$ | H | Cl |
| CN | 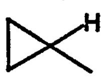 | Br | H | $-SCHF_2$ | H | H |
| CN | 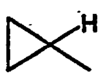 | Br | H | $-SCHF_2$ | H | Br |
| CN | H | Cl | H | $-SCF_2CHF_2$ | H | H |
| CN | H | Cl | H | $-SCF_2CHF_2$ | H | Cl |
| CN | H | Br | H | $-SCF_2CHF_2$ | H | H |
| CN | H | Br | H | $-S-CF_2CHF_2$ | H | Br |
| CN | $CH_3$ | Cl | H | $-SCF_2CHF_2$ | H | H |
| CN | $CH_3$ | Cl | H | $-SCF_2CHF_2$ | H | Cl |
| CN | $CH_3$ | Br | H | $-SCF_2CHF_2$ | H | H |
| CN | $CH_3$ | Br | H | $-SCF_2CHF_2$ | H | Br |
| CN | $C_2H_5$ | Cl | H | $-SCF_2CHF_2$ | H | H |
| CN | $C_2H_5$ | Cl | H | $-S-CF_2CHF_2$ | H | Cl |
| CN | $C_2H_5$ | Br | H | $-SCF_2CHF_2$ | H | H |
| CN | $C_2H_5$ | Br | H | $-SCF_2CHF_2$ | H | Br |
| CN | 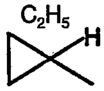 | Cl | H | $-SCF_2CHF_2$ | H | H |
| CN | 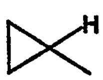 | Cl | H | $-SCF_2CHF_2$ | H | Cl |
| CN | 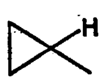 | Br | H | $-SCF_2CHF_2$ | H | H |
| CN | 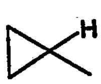 | Br | H | $-SCF_2CHF_2$ | H | Br |
| CN | H | Cl | H | $-SCF_2CHFCl$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | H | Cl | H | $-SCF_2CHFCl$ | H | Cl |
| CN | H | Br | H | $-SCF_2CHFCl$ | H | H |
| CN | H | Br | H | $-SCF_2CHFCl$ | H | Br |
| CN | $CH_3$ | Cl | H | $-SCF_2CHFCl$ | H | H |
| CN | $CH_3$ | Cl | H | $-SCF_2CHFCl$ | H | Cl |
| CN | $CH_3$ | Br | H | $-SCF_2CHFCl$ | H | H |
| CN | $CH_3$ | Br | H | $-SCF_2CHFCL$ | H | Br |
| CN | $C_2H_5$ | Cl | H | $-SCF_2CHFCl$ | H | H |
| CN | $C_2H_5$ | Cl | H | $-SCF_2CHFCl$ | H | Cl |
| CN | $C_2H_5$ | Br | H | $-SCF_2CHFCl$ | H | H |
| CN | $C_2H_5$ | Br | H | $-SCF_2CHFCl$ | H | Br |
| CN | 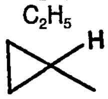 | Cl | H | $-SCF_2CHFCl$ | H | H |
| CN | 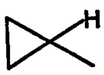 | Cl | H | $-SCF_2CHFCl$ | H | Cl |
| CN | 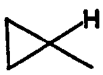 | Br | H | $-SCF_2CHFCl$ | H | H |
| CN | 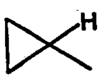 | Br | H | $-SCF_2CHFCl$ | H | Br |
| CN | $ClCH_2-$ | Cl | H | $-SCF_3$ | H | H |
| CN | $ClCH_2-$ | Cl | H | $-OCF_3$ | H | Cl |
| CN | $ClCH_2-$ | Br | H | $-OCF_3$ | H | H |
| CN | $ClCH_2-$ | Br | H | $-OCF_3$ | H | Br |
| CN | $CH_3OCH_2-$ | Cl | H | $-OCF_3$ | H | H |
| CN | $CH_3OCH_2-$ | Cl | H | $-OCF_3$ | H | Cl |
| CN | $ClCH_2-$ | Cl | H | $-SCF_3$ | H | H |
| CN | $ClCH_2-$ | Cl | H | $-SCF_3$ | H | Cl |
| CN | $ClCH_2-$ | Br | H | $-SCF_3$ | H | H |
| CN | H | Cl | H | $-SOCF_3$ | H | H |
| CN | H | Cl | H | $-SOCF_3$ | H | Cl |
| CN | H | Br | H | $-SOCF_3$ | H | Br |
| CN | H | Br | H | $-SOCF_3$ | H | H |
| CN | H | $CF_3$ | H | $-SOCF_3$ | H | H |
| CN | H | $CF_3$ | H | $-SOCF_3$ | H | Cl |
| CN | $CH_3$ | Cl | H | $-SOCF_3$ | H | H |
| CN | $CH_3$ | Cl | H | $-SOCF_3$ | H | Cl |
| CN | $CH_3$ | Br | H | $-SOCF_3$ | H | Br |
| CN | $CH_3$ | Br | H | $-SOCF_3$ | H | H |
| CN | $CH_3$ | $CF_3$ | H | $-SOCF_3$ | H | H |
| CN | $CH_3$ | $CF_3$ | H | $-SOCF_3$ | H | Cl |
| CN | $C_2H_5$ | Cl | H | $-SOCF_3$ | H | H |
| CN | $C_2H_5$ | Cl | H | $-SOCF_3$ | H | Cl |
| CN | $C_2H_5$ | Br | H | $-SOCF_3$ | H | Br |
| CN | $C_2H_5$ | Br | H | $-SOCF_3$ | H | H |
| CN | $C_2H_5$ | $CF_3$ | H | $-SOCF_3$ | H | H |
| CN | $C_2H_5$ | $CF_3$ | H | $-SOCF_3$ | H | Cl |
| CN | 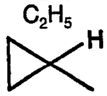 | Cl | H | $-SOCF_3$ | H | H |
| CN | 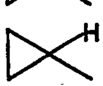 | Cl | H | $-SOCF_3$ | H | Cl |
| CN | 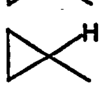 | Br | H | $-SOCF_3$ | H | Br |
| CN | 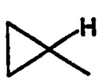 | Br | H | $-SOCF_3$ | H | H |
| CN | 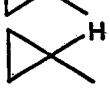 | $CF_3$ | H | $-SOCF_3$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | 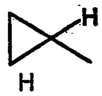 | $CF_3$ | H | $-SOCF_3$ | H | Cl |
| CN | H | Cl | H | $-OCF_2CHFCl$ | H | H |
| CN | H | Cl | H | $-OCF_2CHFCl$ | H | Cl |
| CN | H | Br | H | $-OCF_2CHFCl$ | H | H |
| CN | H | Cl | H | $-OCF_2CHFCl$ | H | Br |
| CN | $CH_3$ | Cl | H | $-OCF_2CHFCl$ | H | H |
| CN | $CH_3$ | Cl | H | $-OCF_2CHFCl$ | H | Cl |
| CN | $CH_3$ | Br | H | $-OCF_2CHFCl$ | H | H |
| CN | $CH_3$ | Br | H | $-OCF_2CHFCl$ | H | Br |
| CN | $C_2H_5$ | Cl | H | $-OCF_2CHFCl$ | H | H |
| CN | $C_2H_5$ | Cl | H | $-OCF_2CHFCl$ | H | Cl |
| CN | $C_2H_5$ | Br | H | $-OCF_2CHFCl$ | H | H |
| CN | $C_2H_5$ | Br | H | $-OCF_2CHFCl$ | H | Br |
| CN | 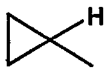 | Cl | H | $-OCF_2CHFCl$ | H | H |
| CN | 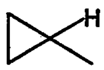 | Cl | H | $-OCF_2CHFCl$ | H | Cl |
| CN | 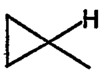 | Br | H | $-OCF_2CHFCl$ | H | H |
| CN | 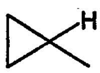 | Br | H | $-OCF_2CHFCl$ | H | Br |
| CN | H | Cl | H | $-OCF_2CHCl_2$ | H | H |
| CN | H | Cl | H | $-OCF_2CHCl_2$ | H | Cl |
| CN | H | Br | H | $-OCF_2CHCl_2$ | H | H |
| CN | H | Br | H | $-OCF_2CHCl_2$ | H | Br |
| CN | $CH_3$ | Cl | H | $-OCF_2CHCl_2$ | H | H |
| CN | $CH_3$ | Cl | H | $-OCF_2CHCl_2$ | H | Cl |
| CN | $CH_3$ | Br | H | $-OCF_2CHCl_2$ | H | H |
| CN | $CH_3$ | Br | H | $-OCF_2CHCl_2$ | H | Br |
| CN | $C_2H_5$ | Cl | H | $-OCF_2CHCl_2$ | H | H |
| CN | $C_2H_5$ | Cl | H | $-OCF_2CHCl_2$ | H | Cl |
| CN | $C_2H_5$ | Br | H | $-OCF_2CHCl_2$ | H | H |
| CN | $C_2H_5$ | Br | H | $-OCF_2CHCl_2$ | H | Br |
| CN | 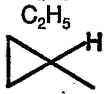 | Cl | H | $-OCF_2CHCl_2$ | H | H |
| CN | 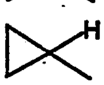 | Cl | H | $-OCF_2CHCl_2$ | H | Cl |
| CN | 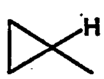 | Br | H | $-OCF_2CHCl_2$ | H | H |
| CN | 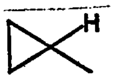 | Br | H | $-OCF_2CHCl_2$ | H | Br |
| CN | H | Cl | H | $-OCF_2CHF_2$ | H | H |
| CN | H | Cl | H | $-OCF_2CHF_2$ | H | Cl |
| CN | H | Br | H | $-OCF_2CHF_2$ | H | H |
| CN | H | Br | H | $-OCF_2CHF_2$ | H | Br |
| CN | $CH_3$ | Cl | H | $-OCF_2CHF_2$ | H | H |
| CN | $CH_3$ | Cl | H | $-OCF_2CHF_2$ | H | Cl |
| CN | $CH_3$ | Br | H | $-OCF_2CHF_2$ | H | H |
| CN | $CH_3$ | Br | H | $-OCF_2CHF_2$ | H | Br |
| CN | $C_2H_5$ | Cl | H | $-OCF_2CHF_2$ | H | H |
| CN | $C_2H_5$ | Cl | H | $-OCF_2CHF_2$ | H | Cl |
| CN | $C_2H_5$ | Br | H | $-OCF_2CHF_2$ | H | H |
| CN | $C_2H_5$ | Br | H | $-OCF_2CHF_2$ | H | Br |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | 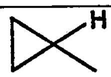 | Cl | H | $-OCF_2CHF_2$ | H | H |
| CN | 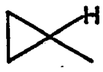 | Cl | H | $-OCF_2CHF_2$ | H | Cl |
| CN | 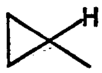 | Br | H | $-OCF_2CHF_2$ | H | H |
| CN | 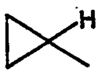 | Br | H | $-OCF_2CHF_2$ | H | Br |
| CN | H | Cl | H | $-SO_2CF_3$ | H | H |
| CN | H | Cl | H | $-SO_2CF_3$ | H | Cl |
| CN | H | Br | H | $-SO_2CF_3$ | H | H |
| CN | H | Br | H | $-SO_2CF_3$ | H | Br |
| CN | H | $CF_3$ | H | $-SO_2CF_3$ | H | H |
| CN | $CH_3$ | $CF_3$ | H | $-SO_2CH_3$ | H | H |
| CN | $C_2H_5$ | Cl | H | $-SO_2CF_3$ | H | Cl |
| CN | $C_2H_5$ | Br | H | $-SO_2CF_3$ | H | H |
| CN | $C_2H_5$ | Br | H | $-SO_2CF_3$ | H | Br |
| CN | $C_2H_5$ | $CF_3$ | H | $-SO_2CF_3$ | H | H |
| CN | 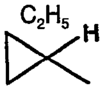 | CL | H | $-SO_2CF_3$ | H | H |
| CN | 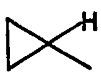 | Cl | H | $-SO_2CF_3$ | H | Cl |
| CN | 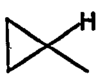 | Br | H | $-SO_2CF_3$ | H | H |
| CN | 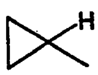 | Br | H | $-SO_2CF_3$ | H | Br |
| CN | 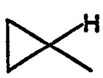 | $CF_3$ | H | $-SO_2CF_3$ | H | H |
| CN | H | F | H | $-SCCl_2F$ | H | H |
| CN | H | F | H | $-SCCl_2F$ | H | F |
| CN | H | F | F | $-SCCl_2F$ | F | F |
| CN | H | Cl | H | $-SCCl_2F$ | H | H |
| CN | H | Cl | H | $-SCCl_2F$ | H | Cl |
| CN | H | Cl | H | $-SCCl_2F$ | H | F |
| CN | H | Br | H | $-SCCl_2F$ | H | H |
| CN | H | Br | H | $-SCCl_2F$ | H | Br |
| CN | $CH_3$ | F | H | $-SCCl_2F$ | H | H |
| CN | $CH_3$ | F | H | $-SCCl_2F$ | H | F |
| CN | $CH_3$ | F | F | $-SCCl_2F$ | F | F |
| CN | $CH_3$ | Cl | H | $-SCCl_2F$ | H | H |
| CN | $CH_3$ | Cl | H | $-SCCl_2F$ | H | Cl |
| CN | $CH_3$ | Cl | H | $-SCCl_2F$ | H | F |
| CN | $CH_3$ | Br | H | $-SCCl_2F$ | H | H |
| CN | $CH_3$ | Br | H | $-SCCl_2F$ | H | Br |
| CN | $C_2H_5$ | F | H | $-SCCl_2F$ | H | H |
| CN | $C_2H_5$ | F | H | $-SCCl_2F$ | H | F |
| CN | $C_2H_5$ | F | H | $-SCCl_2F$ | F | F |
| CN | $C_2H_5$ | Cl | H | $-SCCl_2F$ | H | H |
| CN | $C_2H_5$ | Cl | H | $-SCCl_2F$ | H | Cl |
| CN | $C_2H_5$ | Cl | H | $-SCCl_2F$ | H | F |
| CN | $C_2H_5$ | Br | H | $-SCCl_2F$ | H | H |
| CN | $C_2H_5$ | Br | H | $-SCCl_2F$ | H | Br |
| CN | 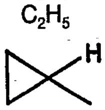 | F | H | $-SCCl_2F$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | 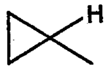 | F | H | $-SCCl_2F$ | H | F |
| CN | 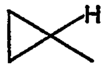 | F | F | $-SCCl_2F$ | F | F |
| CN | 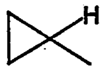 | Cl | H | $-SCCl_2F$ | H | H |
| CN | 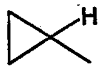 | Cl | H | $-SCCl_2F$ | H | Cl |
| CN | 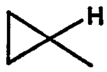 | Cl | H | $-SCCl_2F$ | H | F |
| CN | 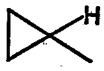 | Br | H | $-SCCl_2F$ | H | H |
| CN | 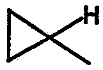 | Br | H | $-SCCl_2F$ | H | Br |
| CN | H | F | H | $-OCHF_2$ | H | H |
| CN | H | F | H | $-OCHF_2$ | H | F |
| CN | H | F | F | $-OCHF_2$ | F | F |
| CN | H | Cl | H | $-OCHF_2$ | H | H |
| CN | H | Cl | H | $-OCHF_2$ | H | Cl |
| CN | H | Cl | H | $-OCHF_2$ | H | F |
| CN | H | Br | H | $-OCHF_2$ | H | H |
| CN | H | Br | H | $-OCHF_2$ | H | Br |
| CN | $CH_3$ | F | H | $-OCHF_2$ | H | H |
| CN | $CH_3$ | F | H | $-OCHF_2$ | H | F |
| CN | $CH_3$ | F | F | $-OCHF_2$ | F | F |
| CN | $CH_3$ | Cl | H | $-OCHF_2$ | H | H |
| CN | $CH_3$ | Cl | H | $-OCHF_2$ | H | Cl |
| CN | $CH_3$ | Cl | H | $-OCHF_2$ | H | F |
| CN | $CH_3$ | Br | H | $-OCHF_2$ | H | H |
| CN | $CH_3$ | Br | H | $-OCHF_2$ | H | Br |
| CN | $C_2H_5$ | F | H | $-OCHF_2$ | H | H |
| CN | $C_2H_5$ | F | H | $-OCHF_2$ | H | F |
| CN | $C_2H_5$ | F | F | $-OCHF_2$ | F | F |
| CN | $C_2H_5$ | Cl | H | $-OCHF_2$ | H | H |
| CN | $C_2H_5$ | Cl | H | $-OCHF_2$ | H | Cl |
| CN | $C_2H_5$ | Cl | H | $-OCHF_2$ | H | F |
| CN | $C_2H_5$ | Br | H | $-OCHF_2$ | H | H |
| CN | $C_2H_5$ | Br | H | $-OCHF_2$ | H | Br |
| CN | 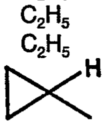 | F | H | $-OCHF_2$ | H | H |
| CN | 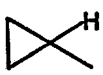 | F | H | $-OCHF_2$ | H | F |
| CN | 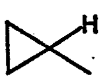 | F | F | $-OCHF_2$ | F | F |
| CN | 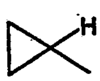 | Cl | H | $-OCHF_2$ | H | H |
| CN | 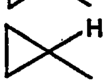 | Cl | H | $-OCHF_2$ | H | Cl |
| CN | 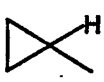 | Cl | H | $-OCHF_2$ | H | F |
| CN | 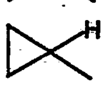 | Br | H | $-OCHF_2$ | H | H |

Tabelle 1 (Fortsetzung)

| $R^1$ | $R^2$ | $R^3$ | $R^4$ | $R^5$ | $R^6$ | $R^7$ |
|---|---|---|---|---|---|---|
| CN | 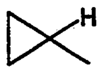 | Br | H | $-OCHF_2$ | H | Br |
| CN | 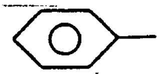 | Cl | H | $-OCF_3$ | H | Cl |
| CN | 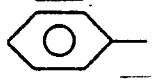 | Cl | H | $-SCF_3$ | H | Cl |
| CN | 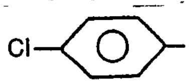 | Cl | H | $-OCF_3$ | H | Cl |
| CN | 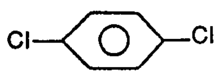 | Cl | H | $-SCF_3$ | H | Cl |
| CN | 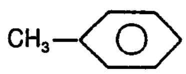 | Cl | H | $-OCF_3$ | H | Cl |
| CN | 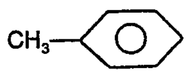 | Cl | H | $-SCF_3$ | H | Cl |
| CN | 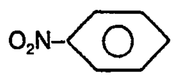 | Cl | H | $-OCF_3$ | H | Cl |
| CN | 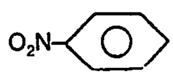 | Cl | H | $-SCF_3$ | H | Cl |

Verwendet man als Ausgangsstoffe beispielsweise 5-Amino-4-cyano-1-(2',6'-dichlor-4'-trifluormethylthio-phenyl)-pyrazol und Propionylchlorid, so lässt sich der Reaktionsablauf des erfindungsgemässen Verfahrens durch das folgende Formelschema darstellen:

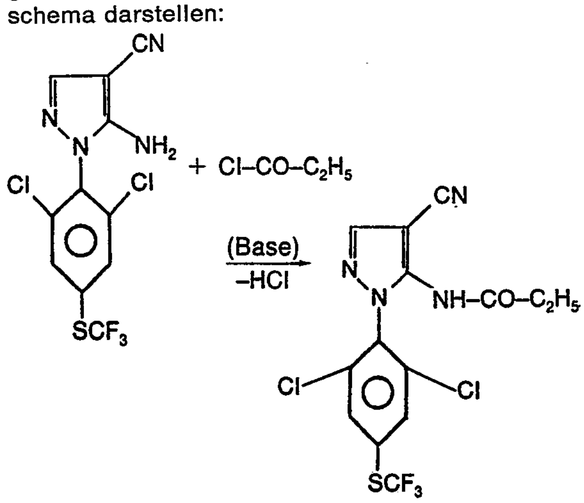


Die zur Durchführung des erfindungsgemässen Verfahrens als Ausgangsstoffe benötigten 5-Aminopyrazole sind durch die Formel (II) allgemein definiert. In dieser Formel (II) besitzen $R^1$, $R^3$, $R^4$, $R^5$, $R^6$ und $R^7$ vorzugsweise diejenigen Bedeutungen, die schon bei der Beschreibung der erfindungsgemässen Stoffe der Formel (I) als bevorzugt für diese Substituenten genannt wurden.

Die 5-Aminopyrazole der Formel (II) sind teilweise bekannt (vergl. z.B. EP 34 945; DE-OS 3 226 496, DE-OS 3 129 429 und DE-OS 3 045 903).

Noch nicht bekannt sind 5-Aminopyrazole der Formel (IIa),

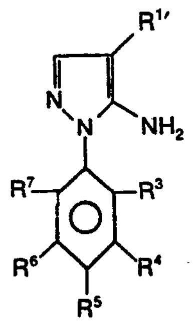

(IIa)

in welcher

$R^{1'}$ für jeweils geradkettiges oder verzweigtes Alkoxycarbonyl, Alkenyloxycarbonyl, Alkinyloxycarbonyl, Alkenylaminocarbonyl oder Alkinylaminocarbonyl mit jeweils bis zu 4 Kohlenstoffatomen in den einzelnen Alkylteilen steht und

$R^3$, $R^4$, $R^5$, $R^6$ und $R^7$ die oben angegebene Bedeutung haben und wobei die Verbindung 5-Amino-4-methoxycarbonyl-1(2-trifluormethyl-4-chlorphenyl)-pyrazol ausgenommen ist.

Man erhält sie jedoch nach prinzipiell bekannten Verfahren, wenn man Acrylnitril-Derivate der Formel (IVa),

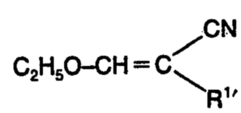

(IVa)

in welcher

$R^{1'}$ die oben angegebene Bedeutung hat,

mit Phenylhydrazinen der Formel (V),

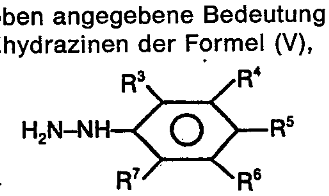

in welcher

$R^3$, $R^4$, $R^5$, $R^6$ und $R^7$ die oben angegebene Bedeutung haben,

entweder zunächst in einer ersten Stufe, gegebenenfalls in Gegenwart eines Verdünnungsmittels, wie beispielsweise Eisessig oder Ethanol, sowie gegebenenfalls in Gegenwart eines Reaktionshilfsmittels, wie beispielsweise Natriumacetat, bei Temperaturen zwischen −20 °C und +20 °C umsetzt zu den Phenylhydrazin-Derivaten der Formel (VIa)

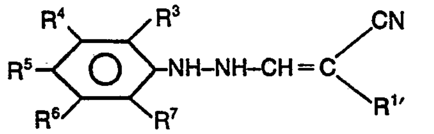
(VIa)

in welcher
$R^{1'}$, $R^3$, $R^4$, $R^5$, $R^6$ und $R^7$ die oben angegebene Bedeutung haben,
und diese in einer zweiten Stufe, gegebenenfalls in Gegenwart eines Verdünnungsmittels wie beispielsweise Ethylenglykolmonoethylether, bei Temperaturen zwischen +50 °C und +150 °C cyclisiert,
oder direkt in einem Reaktionsschritt ohne Isolierung der Zwischenstufe der Formel (VIa), gegebenenfalls in Gegenwart eines Verdünnungsmittels wie beispielsweise Ethylenglykolmonoethylether oder Ethanol bei Temperaturen zwischen +50 °C und +150 °C cyclisiert.

Ebenfalls noch nicht bekannt sind 5-Aminopyrazole der Formel (IIb),

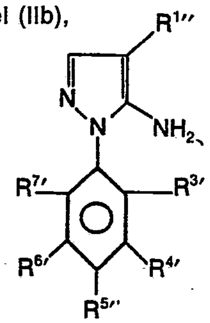
(IIb)

in welcher
$R^{1''}$ für Cyano oder für Alkylaminocarbonyl mit bis zu 4 Kohlenstoffatomen im Alkylteil steht und
$R^{3'}$, $R^{4'}$, $R^{5'}$, $R^{6'}$ und $R^{7'}$, welche gleich oder verschieden sind, für Wasserstoff, Fluor, Chlor, Brom, Iod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für den Rest $-X-R^8$ stehen, wobei X für Sauerstoff, Schwefel, Sulfinyl oder Sulfonyl steht und $R^8$ für Halogenalkyl mit bis zu 4 Kohlenstoffatomen und bis zu 9 gleichen oder verschiedenen Halogenatomen steht mit der Massgabe, dass mindestens einer der Reste $R^{3'}$, $R^{4'}$, $R^{5'}$, $R^{6'}$ oder $R^{7'}$ für einen Rest $-X-R^8$ steht, wobei nicht gleichzeitig X für Sauerstoff und $R^8$ für Trifluormethyl stehen.

Man erhält sie ebenfalls nach prinzipiell bekannten Verfahren in analoger Weise wie die 5-Aminopyrazole der Formel (IIa), wenn man Acrylnitril-Derivate der Formel (IVb),

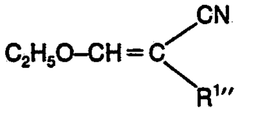
(IVb)

in welcher
$R^{1''}$ die oben angegebene Bedeutung hat,
mit Phenylhydrazinen der Formel (Va),

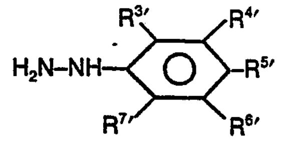
(Va)

in welcher
$R^{3'}$, $R^{4'}$, $R^{5'}$, $R^{6'}$ und $R^{7'}$ die oben angegebene Bedeutung haben,
entweder zunächst in einer ersten Stufe, gegebenenfalls in Gegenwart eines Verdünnungsmittels wie beispielsweise Eisessig oder Ethanol, und gegebenenfalls in Gegenwart eines Reaktionshilfsmittels, wie beispielsweise Natriumacetat, bei Temperaturen zwischen −20 °C und +20 °C umsetzt zu den Phenylhydrazin-Derivaten der Formel (VIb),

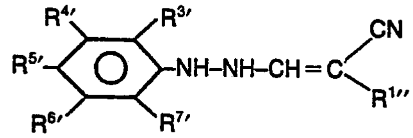
(VIb)

in welcher
$R^{1''}$, $R^{3'}$, $R^{4'}$, $R^{5'}$, $R^{6'}$ und $R^{7'}$ die oben angegebene Bedeutung haben,
und diese in einer 2. Stufe, gegebenenfalls in Gegenwart eines Verdünnungsmittels, wie beispielsweise Ethylenglykolmonoethylether bei Temperaturen zwischen +50 °C und +150 °C cyclisiert,
oder direkt in einem Reaktionsschritt ohne Isolierung der Zwischenstufe der Formel (IVb), gegebenenfalls in Gegenwart eines Verdünnungsmittels, wie beispielsweise Ethylenglykolmonoethylether oder Ethanol bei Temperaturen zwischen +50 °C und +150 °C cyclisiert.

Die Acrylnitril-Derivate der Formeln (IVa) und (IVb) sind bekannt (vergl. z.B. EP 34 945 oder DE-OS 3 129 429).

Die Phenylhydrazine der Formeln (V) und (Va) sind grösstenteils bekannt oder können nach bekannten Verfahren in einfacher, analoger Art und Weise hergestellt werden (vergl. z.B. Houben-Weyl, ‹Methoden der organischen Chemie› Band X/2, S. 203, Thieme Verlag Stuttgart, 1967), indem man beispielsweise die bekannten Aniline der Formel (VII),

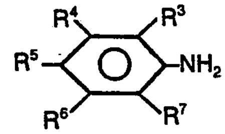
(VII)

in welcher
$R^3$, $R^4$, $R^5$, $R^6$ oder $R^7$ die oben angegebene Bedeutung haben, mit Natriumnitrit in Gegenwart einer Säure, wie beispielsweise Schwefelsäure, und dann mit Zinn-II-chlorid ebenfalls in Gegenwart einer Säure, wie beispielsweise Salzsäure, bei Temperaturen zwischen −20 °C und +80 °C umsetzt.

Die weiterhin zur Durchführung des erfindungsgemässen Verfahrens als Ausgangsstoffe benötigten Acylierungsmittel sind durch die Formel (III)

allgemein definiert. In dieser Formel (III) besitzt $R^2$ vorzugsweise diejenigen Bedeutungen, die schon bei der Beschreibung der erfindungsgemässen Stoffe der Formel (I) als bevorzugt für diesen Rest genannt wurden. A steht vorzugsweise für Halogen, insbesondere Chlor oder Brom, oder für einen Rest $R^2$–CO–O–, wobei $R^2$ die oben angegebene Bedeutung hat. Die Acylierungsmittel der Formel (III) sind allgemein bekannte Verbindungen der organischen Chemie.

Als Verdünnungsmittel zur Durchführung des erfindungsgemässen Verfahrens kommen inerte organische Lösungsmittel infrage. Vorzugsweise verwendet man aliphatische oder aromatische, gegebenenfalls halogenierte Kohlenwasserstoffe, wie Benzin, Benzol, Toluol, Xylol, Pentan, Hexan, Heptan, Cyclohexan, Petrolether, Ligroin, Methylenchlorid, Ethylenchlorid, Chloroform, Tetrachlorkohlenstoff, Chlorbenzol oder Dichlorbenzol, Ether, wie Diethylether oder Diisopropylether, Ethylenglykoldimethylether, Tetrahydrofuran oder Dioxan, Ketone, wie Aceton oder Butanon, Methylisopropylketon oder Methylisobutylketon, Ester, wie Essigsäureethylester, Nitrile, wie Acetonitril oder Propionitril, Amide, wie Dimethylformamid, Dimethylacetamid, N-Methylpyrrolidon oder Hexamethylphosphorsäuretriamid.

Verwendet man Acylierungsmittel der Formel (III) in flüssiger Form, so ist es auch möglich, diese in entsprechendem Überschuss als Verdünnungsmittel einzusetzen.

Als Säurebindemittel kommen für das erfindungsgemässe Verfahren alle üblicherweise verwendbaren anorganischen und organischen Basen infrage. Vorzugsweise verwendet man Alkalimetallhydroxide oder -carbonate, wie beispielsweise Natriumhydroxid, Kaliumhydroxid, Natriumcarbonat oder Kaliumcarbonat oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei dem erfindungsgemässen Verfahren in einem grösseren Bereich variiert werden. Im allgemeinen arbeitet man zwischen –20 °C und +150 °C, vorzugsweise zwischen 0 °C und +100 °C.

Zur Durchführung des erfindungsgemässen Verfahrens setzt man pro Mol 5-Aminopyrazol der Formel (II) im allgemeinen 1 bis 20 Mol, vorzugsweise 1 bis 15 Mol an Acylierungsmittel der Formel (III) und im allgemeinen 1 bis 3 Mol, vorzugsweise 1 bis 2 Mol an Säurebindemittel ein. Die Reaktionsführung, Aufarbeitung und Isolierung der Endprodukte der Formel (I) erfolgt in üblicher Art und Weise.

Die erfindungsgemässen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen:-
Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen:
Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:
Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:
Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemässen Wirkstoffe der Formel (I) neben einer besonders guten allgemeinen herbiziden Wirksamkeit auch eine deutlich verbesserte Kulturpflanzenselektivität in wichtigen Kulturen.

Die Vorprodukte der Formel (II) ebenso wie die Vorprodukte der Formeln (VIa) und (VIb) besitzen ebenfalls herbizide Wirkungen und ausgeprägte Selektivität gegenüber wichtigen Kulturpflanzen.

In entsprechenden Aufwandmengen besitzen die erfindungsgemässen Wirkstoffe der Formel (I) ebenso wie deren Vorprodukte der Formel (II) auch eine fungizide Wirkung, beispielsweise gegen den Erreger der Reiskrankheit Pyricularia oryzae.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemässen Wirkstoffe können als solche oder in ihren Formulierungen auch in Mischungen mit bekannten Herbiziden zur Unkrautbekämpfung Verwendung finden, wobei Fertigformulierung oder Tankmischung möglich sind.

Für die Mischungen kommen bekannte Herbizide, beispielsweise Diphenylether, Pyridoxyphenoxypropionsäure, Phenoxyalkancarbonsäuren, Harnstoffe, Triazinone oder Triazindione, wie z.B.

α-[4-(3,5-Dichlor-2-pyridoxy)-phenoxy]-propionsäure-2-benzyloxyethylester,
-2,2-diethoxyethylester oder -trimethyl-silylmethylester,
2,4-Dichlorphenoxyessigsäure,
α-(2,4-Dichlorphenoxy)-propionsäure,
4-Chlor-2-methylphenoxy-essigsäure,
α-(4-Chlor-2-methylphenoxy)-propionsäure,
1-Amino-6-ethylthio-3-(2,2-dimethylpropyl)-1,3,5-triazin-2,4(1H,3H)-dion oder
N-(2-Benzthiazolyl)-N,N'-dimethyl-harnstoff
zur Unkrautbekämpfung in Getreide;
4-Amino-3-methyl-6-phenyl-1,2,4-triazin-5(4H)-on
zur Unkrautbekämpfung in Zuckerrüben und
4-Amino-6-(1,1-dimethylethyl)-3-methylthio- oder -3-ethylthio-1,2,4-triazin-5(4H)-on
zur Unkrautbekämpfung in Sojabohnen infrage.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem grösseren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemässen Wirkstoffe geht aus den folgenden Beispielen hervor.

Herstellungsbeispiele

Beispiel 1

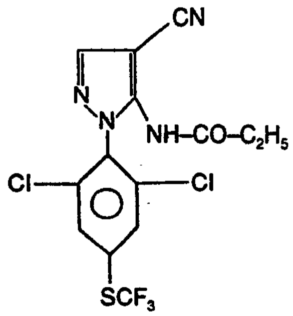

(1)

Eine Suspension von 3,5 g (0,01 Mol)
5-Amino-4-cyano-1-(2,6-dichlor-4-trifluormethylthio-phenyl)-pyrazol
in 30 ml Chloroform werden unter Rühren bei 0 °C zunächst mit 10 ml (0,11 Mol) Propionylchlorid und danach mit 1,8 ml (0,02 Mol) Pyridin in 15 ml Chloroform versetzt. Man erhält eine klare Lösung, die nach beendeter Zugabe weitere 20 Stunden bei Raumtemperatur gerührt wird. Die so erhaltene Lösung wird zur Trockne eingedampft. Zur Aufarbeitung nimmt man in 50 ml Ethanol auf, gibt wässrigen Ammoniak zu bis zur alkalischen Reaktion, erhitzt 10 Minuten am Rückfluss, entfernt die flüchtigen Bestandteile im Vakuum, nimmt den Rückstand in 100 ml Chloroform auf, wäscht mit Wasser, dann mit 2N wässriger Salzsäure und wieder mit Wasser, trocknet über Natriumsulfat und entfernt das Lösungsmittel im Vakuum. Man erhält 3,4 g (83% der Theorie) an
5-Propionyl-amino-4-cyano-1-(2,6-dichlor-4-trifluormethylthio-phenyl)-pyrazol
vom Schmelzpunkt 153 °C bis 156 °C.

Beispiel 2

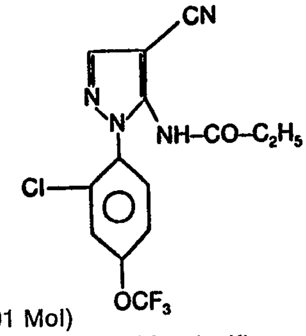

2,9 g (0,01 Mol)
5-Amino-4-cyano-1-(2-chlor-4-trifluormethoxy-phenyl)-pyrazol
in 30 ml Chloroform werden bei 0 °C unter Rühren mit 10 ml (0,11 Mol) Propionylchlorid und danach ebenfalls bei 0 °C mit einer Lösung von 1,8 ml (0,02 Mol) Pyridin in 15 ml Chloroform versetzt. Nach beendeter Zugabe rührt man weitere 20 Stunden bei Raumtemperatur, zieht das Lösungsmittel ab, nimmt den Rückstand in 50 ml Ethanol auf, gibt wässrigen Ammoniak zu bis zur alkalischen Reaktion, entfernt die flüchtigen Bestandteile im Vakuum, nimmt den Rückstand in 100 ml Chloroform auf, wäscht mit Wasser, dann mit 2N wässriger Salzsäure und wieder mit Wasser, trocknet über Natriumsulfat und entfernt das Lösungsmittel im Vakuum. Man erhält 2,7 g (75,3% der Theorie) an
4-Cyano-1-(2-chlor-4-trifluormethoxy-phenyl)-5-propionylamino-pyrazol
vom Schmelzpunkt 127 °C.

In entsprechender Weise und gemäss den allgemeinen Herstellungsangaben erhält man die folgenden Verbindungen der allgemeinen Formel (I):

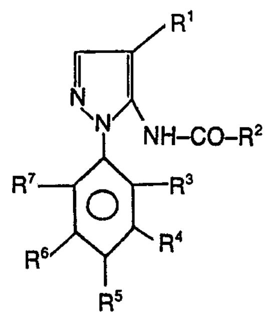

(I)

Tabelle 2

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 3 | $C_2H_5OCO-$ | $C_2H_5$ | Cl | H | $CF_3$ | H | Cl | 63–67 |
| 4 | $C_2H_5OCO-$ | $C_2H_5$ | Cl | H | $CF_3$ | H | H | 87–89 |
| 5 | CN | $C_2H_5$ | H | Cl | $OCF_3$ | H | H | 142–143 |
| 6 | CN | $C_2H_5$ | H | H | $OCF_3$ | H | H | 152–153 |
| 7 | $C_2H_5OCO-$ | $C_2H_5$ | Cl | H | $OCF_3$ | H | Cl | 84–85 |
| 8 | $C_2H_5OCO-$ | $C_2H_5$ | Cl | H | $OCF_3$ | H | H | Oel $n_D^{20} = 1,507$ |
| 9 | CN | $C_2H_5$ | H | H | $SCF_3$ | H | H | 171 |
| 10 | CN | $C_2H_5$ | H | Cl | $SCF_3$ | H | H | 143 |
| 11 | CN | $C_2H_5$ | Cl | H | $SCF_3$ | H | H | 161–163 |
| 12 | CN | $CH_3$ | Cl | H | $SCF_3$ | H | Cl | 173–175 |
| 13 | $C_2H_5OCO-$ | $C_2H_5$ | H | Cl | $SCF_3$ | H | H | 136–137 |
| 14 | CN | $C_2H_5$ | $CF_3$ | H | $CF_3$ | H | H | 150–154 |

Tabelle 2 (Fortsetzung)

| Bsp. Nr. | $R^1$ | $R^2$ | $R^3$ | $R^4$ | $R^5$ | $R^6$ | $R^7$ | Schmelzpunkt (°C) |
|---|---|---|---|---|---|---|---|---|
| 15 | CN | $C_2H_5$ | $CF_3$ | H | $F_3C-SO_2-$ | H | H | 188–190 |
| 16 | CN | $C_2H_5$ | Cl | H | $F_3C-SO_2-$ | H | H | 160 |
| 17 | CN | $C_2H_5$ | H | $CF_3$ | Cl | H | H | 186 |
| 18 | CN | $C_2H_5$ | $CF_3$ | H | $CH_3-SO_2-$ | H | H | 148 |
| 19 | CN | $C_2H_5$ | $CF_3$ | H | $CH_3-SO_2-$ | H | Cl | 198 |
| 20 | CN | $C_2H_5$ | Cl | H | $F_3C-SO_2-$ | H | Cl | 197 |
| 21 | CN | $Cl-CH_2-$ | Cl | H | $F_3CS-$ | H | Cl | 79 |
| 22 | CN | $C_2H_5$ | Cl | H | $ClF_2C-SO_2-$ | H | Cl | 213–214 |
| 23 | $H_2N-\overset{O}{\overset{\|}{C}}-$ | $C_2H_5$ | Cl | H | $F_3C-S-$ | H | Cl | 170–171 |
| 24 | CN | $C_2H_5$ | $CF_3$ | H | H | H | $CF_3$ | 205 |
| 25 | CN | 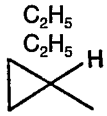 | Cl | H | $F_3C-S-$ | H | Cl | 209 |
| 26 | CN | $Cl-CH_2-$ | Cl | H | $F_3C-O-$ | H | H | 125–128 |
| 27 | CN | 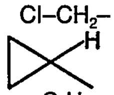 | Cl | H | $F_3C-O-$ | H | H | 116 |
| 28 | CN | $n-C_3H_7$ | Cl | H | $F_3C-O-$ | H | H | 111 |
| 29 | CN | $(CH_2)_2CH-CH_2-$ | Cl | H | $F_3C-O-$ | H | H | 95–98 |
| 30 | CN | $Cl-CH_2-CH_2-$ | Cl | H | $F_3C-O-$ | H | H | 109 |
| 31 | CN | $Br-\underset{CH_3}{\overset{CH_3}{C}}-$ | Cl | H | $F_3C-O-$ | H | H | 120 |
| 32 | CN | $(CH_3)_3C-$ | Cl | H | $F_3C-O-$ | H | H | 159 |
| 33 | CN | $CH_3-(CH_2)_{10}-$ | Cl | H | $F_3C-O-$ | H | H | 70–72 |
| 34 | CN | $CH_3-(CH_2)_3-$ | Cl | H | $F_3C-O-$ | H | H | 84–87 |
| 35 | CN | $CH_3$ | Cl | H | $F_3C-O-$ | H | H | 145–146 |
| 36 | CN | $C_2H_5-\underset{CH_3}{CH}-$ | Cl | H | $F_3C-O-$ | H | H | 107–109 |
| 37 | CN | $CH_3$ | Cl | H | $F_3C-O-$ | H | Cl | 181–182 |
| 38 | CN | $C_2H_5$ | Cl | H | $F_3C-O-$ | H | Cl | |
| 39 | CN | $(CH_3)_2CH-$ | Cl | H | $F_3C-O-$ | H | H | 125–129 |
| 40 | CN | $CH_3O-CH_2-$ | Cl | H | $F_3C-O-$ | H | H | 102 |
| 41 | CN | $CH_3S-CH_2-$ | Cl | H | $F_3C-S-$ | H | Cl | 132–135 |
| 42 | CN | $CH_3O-CH_2-$ | Cl | H | $F_3C-S-$ | H | Cl | 158–160 |
| 43 | CN | $CH_3S-CH_2-$ | Cl | H | $F_3C-O-$ | H | H | 115 |

Herstellung der Ausgangsstoffe:

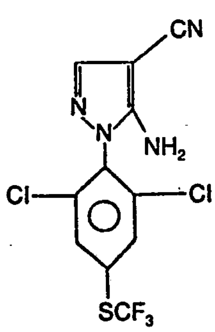

(II–1)

14,1 g (0,04 Mol)
1-(2,2-Dicyanethen-1-yl)-2-(2,6-dichlor-4-trifluormethylthio-phenyl)-hydrazin
in 30 ml Ethylenglykolmonoethylether werden 2 Stunden unter Rückfluss erhitzt. Die heisse Lösung wird mit Aktivkohle versetzt, filtriert und mit 60 ml Wasser verdünnt. Der ausgefallene Niederschlag wird abgesaugt und getrocknet. Man erhält 9,8 g (70% der Theorie) an
5-Amino-4-cyano-1-(2,6-dichlor-4-trifluormethylthiophenyl)-pyrazol
vom Schmelzpunkt 185 °C bis 187 °C.

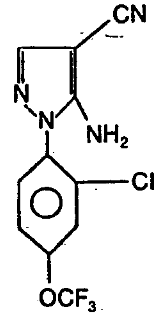

(II–2)

3,08 g (0,025 Mol) Ethoxymethylenmalonsäuredinitril und 5,7 g (0,025 Mol) 2-Chlor-4-trifluormethoxy-phenyl hydrazin in 50 ml Ethylenglykolmonoethylether werden 3 Stunden unter Rückfluss erhitzt, nach dem Abkühlen auf Wasser gegossen, der kristalline Niederschlag abgesaugt, mit Petrolether verrührt, gekühlt und abermals abgesaugt. Man erhält 5,3 g (73,6% der Theorie) an 5-Amino-4-cyano-1-(2-chlor-4-trifluormethoxy-phenyl)pyrazol vom Schmelzpunkt 115 °C.

In entsprechender Weise und entsprechend den allgemeinen Herstellungsangaben erhält man die folgenden neuen 5-Aminopyrazole der Formel (II):

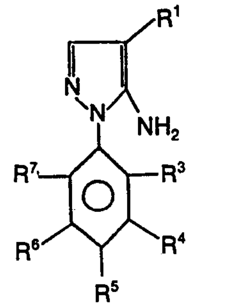

(II)

Tabelle 3

| Bsp. Nr. | $R^1$ | $R^3$ | $R^4$ | $R^5$ | $R^6$ | $R^7$ | Schmelzpunkt (°C) |
|---|---|---|---|---|---|---|---|
| II-3 | $C_2H_5OCO-$ | Cl | H | $CF_3$ | H | Cl | 134-35 |
| II-4 | $C_2H_5OCO-$ | Cl | H | $CF_3$ | H | H | 112-114 |
| II-5 | $C_2H_5OCO-$ | Cl | H | $OCF_3$ | H | H | 115-118 |
| II-6 | $C_2H_5OCO-$ | Cl | H | $OCF_3$ | H | Cl | 153-154 |
| II-7 | CN | Cl | H | $SCF_3$ | H | H | 159 |
| II-8 | CN | H | Cl | $SCF_3$ | H | H | 126-128 |
| II-9 | CN | H | H | $SCF_3$ | H | H | 122 |
| II-10 | $C_2H_5OCO-$ | H | Cl | $SCF_3$ | H | H | 120-122 |
| II-11 | $C_2H_5OCO-$ | Cl | H | $SCF_3$ | H | H | 129-130 |
| II-12 | CN | $CF_3$ | H | $CF_3$ | H | H | 173 |
| II-13 | CN | $CF_3$ | H | $CH_3-SO_2-$ | H | | 188-191 |
| II-14 | CN | $CF_3$ | H | $F_3C-SO_2-$ | H | H | 174-176 |
| II-15 | CN | H | $CF_3$ | Cl | H | H | 118 |
| II-16 | CN | Cl | H | $F_3C-SO_2-$ | H | H | 187-189 |
| II-17 | CN | $CF_3$ | H | $CH_3-SO_2-$ | H | Cl | 181 |
| II-18 | CN | Cl | H | $F_3C-SO_2-$ | H | Cl | 254 |
| II-19 | CN | Cl | H | $ClF_2C-SO_2-$ | H | Cl | 211 |
| II-20 | CN | $CF_3$ | H | H | H | $CF_3$ | 200-203 |
| II-21 | CN | Cl | H | $F_3-C-O-$ | H | Cl | 171 |

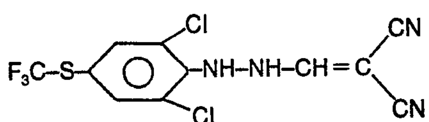

(VI-1)

Zu einer Suspension von 13,9 g (0,05 Mol) (2,6-Dichlor-4-trifluormethylthio)-phenylhydrazin und 2,1 g (0,025 Mol) Natriumacetat in 25 ml Eisessig gibt man unter Rühren 6,1 g (0,05 Mol) Ethoxymethylenmalonsäurenitril. Nach beendeter Zugabe rührt man eine weitere Stunde bei Raumtemperatur und filtriert den so erhaltenen Feststoff ab, welcher nacheinander mit Wasser, wässriger Natriumhydrogencarbonatlösung und wieder mit Wasser gewaschen und anschliessend getrocknet wird. Man erhält 15,8 g (89% der Theorie) an 1-(2,2-Dicyanethen-1-yl)-2-(2,6-dichlor-4-trifluormethyl-thiophenyl)hydrazin vom Schmelzpunkt 160 °C.

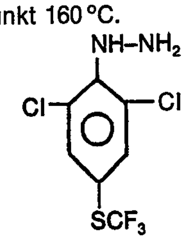

(V-1)

50 g (0,2 Mol) 2,6-Dichlor-4-trifluormethylthio-anilin in 435 ml Eisessig werden bei 55 °C bis 60 °C zunächst mit 16,6 g (0,24 Mol) Natriumnitrit in 150 ml konzentrierter Schwefelsäure und danach bei 5 °C bis 10 °C mit 180,5 g (0,8 Mol) Zinn-II-chloriddihydrat in 188 ml konzentrierter Salzsäure versetzt. Der so erhaltene Niederschlag wird abgesaugt, in 650 ml eines Gemisches aus Eis und wässriger Ammoniaklösung verrührt, abgesaugt, getrocknet, zweimal mit je einem Liter Chloroform ausgekocht, filtriert und das Filtrat im Vakuum vom Lösungsmittel befreit. Man erhält 33 g (62,4% der Theorie) an (2,6-Dichlor-4-trifluormethylthio)-phenylhydrazin vom Schmelzpunkt 58 °C.

In entsprechender Weise und gemäss den allgemeinen Herstellungsangaben erhält man die folgenden neuen Zwischenprodukte der Formel (V):

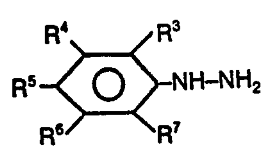

(V)

Tabelle 4

| Bsp. Nr. | $R^3$ | $R^4$ | $R^5$ | $R^6$ | $R^7$ | Schmelzpunkt (°C) |
|---|---|---|---|---|---|---|
| V–2 | Cl | H | $OCF_3$ | H | H | 35 |
| V–3 | Cl | H | $OCF_3$ | H | Cl | 61 |
| V–4 | H | Cl | $OCF_3$ | H | H | 41–42 |
| V–5 | H | H | $OCF_3$ | H | H | Oel, $n_D^{20} = 1,4799$ |
| V–6 | H | H | $SCF_3$ | H | H | 55 |
| V–7 | H | Cl | $SCF_3$ | H | H | 72 |
| V–8 | Cl | H | $SCF_3$ | H | H | 83 |

Anwendungsbeispiele:
In den folgenden Anwendungsbeispielen wird die nachstehend aufgeführte Verbindung als Vergleichssubstanz eingesetzt:

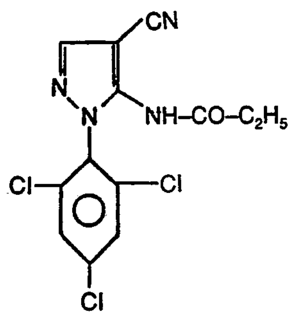
(A)

4-Cyano-5-propionylamino-1-(2,4,6-trichlorphenyl)-pyrazol (bekannt aus DE-OS 3 226 513).

Beispiel A
Pre-emergence-Test
Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalem Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Beispiel zeigt z.B. die Verbindung gemäss Herstellungsbeispiel (1) eine deutliche Überlegenheit in der herbiziden Wirksamkeit ebenso wie in der Nutzpflanzenselektivität im Vergleich zum Stand der Technik; dies gilt insbesondere für Weizen.

Beispiel B
Post-emergence-Test
Lösungsmittel: 5 Gewichtsteile Aceton
Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

In diesem Beispiel zeigt z.B. die Verbindung gemäss Herstellungsbeispiel (1) eine deutliche Überlegenheit in der herbiziden Wirksamkeit, ebenso wie in der Nutzpflanzenselektivität im Vergleich zum Stand der Technik. Dies gilt ebenfalls insbesondere für Weizen.

**Patentansprüche**

1. Substituierte 5-Acylamino-1-phenylpyrazole der allgemeinen Formel (I),

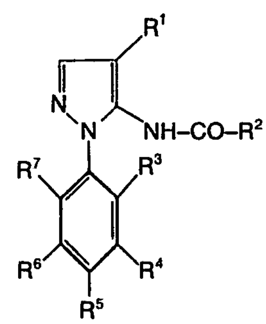
(I)

in welcher

$R^1$ für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl oder Alkinylaminocarbonyl mit jeweils bis zu 4 Kohlenstoffatomen in den einzelnen Alkylteilen steht,

$R^2$ für Wasserstoff, für jeweils geradkettiges oder verzweigtes Alkyl, Alkenyl oder Alkinyl, mit bis zu 6 Kohlenstoffatomen, für Cycloalkyl mit 3 bis 7 Kohlenstoffatomen, für jeweils geradkettiges oder verzweigtes Alkoxyalkyl oder Alkylthioalkyl mit jeweils bis zu 4 Kohlenstoffatomen in den einzelnen Alkylteilen oder für geradkettiges oder verzweigtes Halogenalkyl mit bis zu 6 Kohlenstoffatomen und bis zu 9 gleichen oder verschiedenen Halogenatomen, oder für einfach oder mehrfach, gleich oder verschieden substituiertes Phenyl steht, wobei als Substituenten in Frage kommen: Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen, und

$R^3$, $R^4$, $R^5$, $R^6$ und $R^7$ unabhängig voneinander für Wasserstoff, Fluor, Chlor, Brom, Jod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X)_n-R^8$ stehen, wobei

X für Sauerstoff, Schwefel, Sulfinyl oder Sulfonyl steht,

n für 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$, $R^5$, $R^6$ oder $R^7$ für einen Rest $-(X)_n-R^8$ steht, wobei jedoch nicht gleichzeitig $R^1$ für Cyano und $R^5$ für Trifluormethyl stehen.

2. 5-Propionylamino-4-cyano-1-(2,6-dichlor-4-trifluormethylthio-phenyl)-pyrazol (1) gemäss Anspruch 1.

3. 5-Propionylamino-4-cyano-1-(2,4-di-(trifluormethyl)-phenyl-pyrazol.

4. Verfahren zur Herstellung von substituierten 5-Acylamino-1-phenylpyrazolen der allgemeinen Formel (I)

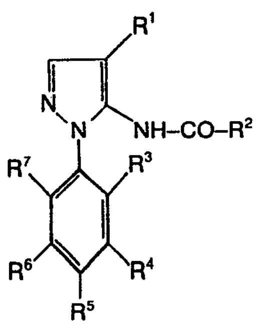

(I)

in welcher

$R^1$ für Cyano, für Aminocarbonyl oder für jeweils geradkettiges oder verzweigtes Alkoxycarbonyl, Alkenyloxycarbonyl, Alkinyloxycarbonyl, Alkylaminocarbonyl, Alkenylaminocarbonyl oder Alkinylaminocarbonyl mit jeweils bis zu 4 Kohlenstoffatomen in den einzelnen Alkylteilen steht,

$R^2$ für Wasserstoff, für jeweils geradkettiges oder verzweigtes Alkyl, Alkenyl oder Alkinyl, mit bis zu 6 Kohlenstoffatomen, für Cycloalkyl mit 3 bis 7 Kohlenstoffatomen, für jeweils geradkettiges oder verzweigtes Alkoxyalkyl oder Alkylthioalkyl mit jeweils bis zu 4 Kohlenstoffatomen in den einzelnen Alkylteilen oder für geradkettiges oder verzweigtes Halogenalkyl mit bis zu 6 Kohlenstoffatomen und bis zu 9 gleichen oder verschiedenen Halogenatomen, oder für einfach oder mehrfach, gleich oder verschieden substituiertes Phenyl steht, wobei als Substituenten in Frage kommen: Halogen, Cyano, Nitro, jeweils geradkettiges oder verzweigtes Alkyl, Alkoxy, Alkylthio oder Alkoxycarbonyl mit jeweils bis zu 4 Kohlenstoffatomen in den einzelnen Alkylteilen, und

$R^3$, $R^4$, $R^5$, $R^6$ und $R^7$ unabhängig voneinander für Wasserstoff, Fluor, Chlor, Brom, Jod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für einen Rest $-(X)_n-R^8$ stehen, wobei

X für Sauerstoff, Schwefel, Sulfinyl oder Sulfonyl steht,

n für 0 oder 1 steht und

$R^8$ für geradkettiges oder verzweigtes Halogenalkyl mit bis zu 4 Kohlenstoffatomen und bis zu 9 gleichen oder verschiedenen Halogenatomen steht, mit der Massgabe, dass mindestens einer der Reste $R^3$, $R^4$, $R^5$, $R^6$ oder $R^7$ für einen Rest $-(X)_n-R^8$ steht, wobei jedoch nicht gleichzeitig $R^1$ für Cyano und $R^5$ für Trifluormethyl stehen, dadurch gekennzeichnet, dass man 5-Aminopyrazole der Formel (II)

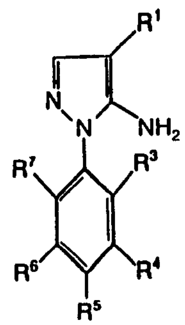

(II)

in welcher

$R^1$, $R^3$, $R^4$, $R^5$, $R^6$ und $R^7$ die oben angegebene Bedeutung haben,

mit Acylierungsmitteln der Formel (III)

$$R^2-CO-A \qquad (III),$$

in welcher

$R^2$ die oben angegebene Bedeutung hat und

A für eine aktivierende Abgangsgruppe steht, gegebenenfalls in Gegenwart eines Verdünnungsmittels und gegebenenfalls in Gegenwart eines Säurebindemittels umsetzt.

5. Herbizide Mittel, gekennzeichnet durch einen Gehalt an mindestens einem substituierten 5-Acylamino-1-phenylpyrazol der Formel (I) gemäss Anspruch 1 und 4 und der Verbindung gemäss Anspruch 3.

6. Verwendung von substituierten 5-Acylamino-1-phenylpyrazolen der Formel (I) gemäss Anspruch 1 und 4 und der Verbindung gemäss Anspruch 3 zur Bekämpfung von unerwünschtem Pflanzenwachstum.

7. Verfahren zur Herstellung von herbiziden Mitteln, dadurch gekennzeichnet, dass man substituierte 5-Acylamino-1-phenylpyrazole der Formel (I) gemäss Anspruch 1 und 4 und die Verbindung gemäss Anspruch 3 mit Streckmitteln und/oder oberflächenaktiven Mitteln vermischt.

8. 5-Aminopyrazole der Formel (IIa)

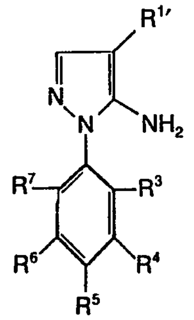

(IIa)

in welcher

$R^{1'}$ für jeweils geradkettiges oder verzweigtes Alkoxycarbonyl, Alkenyloxycarbonyl, Alkinyloxycarbonyl, Alkenylaminocarbonyl oder Alkinylaminocarbonyl mit jeweils bis zu 4 Kohlenstoffatomen in den einzelnen Alkylteilen steht,

$R^3$, $R^4$, $R^5$, $R^6$ und $R^7$ die in Anspruch 1 angegebene Bedeutung haben und

wobei die Verbindung

5-Amino-4-methoxycarbonyl-1-(2-trifluormethyl-4-chlor-phenyl)-pyrazol

ausgenommen ist.

9. 5-Aminopyrazole der Formel (IIb)

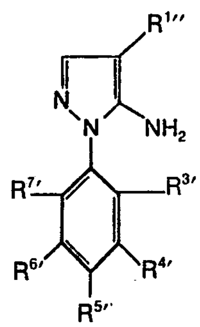

(IIb)

in welcher

$R^{1''}$ für Cyano oder für Alkylaminocarbonyl mit bis zu 4 Kohlenstoffatomen im Alkylteil steht und

$R^{3'}$, $R^{4'}$, $R^{5'}$, $R^{6'}$ und $R^{7'}$, welche gleich oder verschieden sind, für Wasserstoff, Fluor, Chlor, Brom, Jod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für den Rest $-X-R^8$ stehen, wobei X für Sauerstoff, Schwefel, Sulfinyl oder Sulfonyl steht und $R^8$ für Halogenalkyl mit bis zu 4 Kohlenstoffatomen und bis zu 9 gleichen oder verschiedenen Halogenatomen steht mit der Massgabe, dass mindestens einer der Reste $R^{3'}$, $R^{4'}$, $R^{5'}$, $R^{6'}$ oder $R^{7'}$ für einen Rest $-X-R^8$ steht, wobei nicht gleichzeitig X für Sauerstoff und $R^8$ für Trifluormethyl stehen.

10. Phenylhydrazin-Derivate der Formel (VIa)

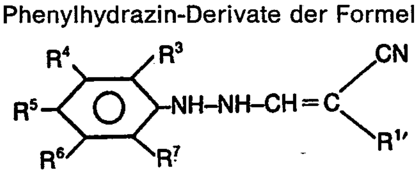

(VIa)

in welcher

$R^1$, $R^3$, $R^4$, $R^5$, $R^6$ und $R^7$ die in Anspruch 8 bzw. 1 angegebene Bedeutung haben.

11. Phenylhydrazin-Derivate der Formel (VIb)

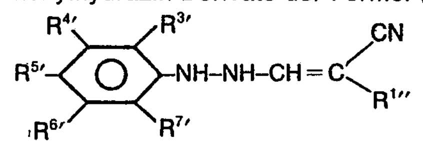

(VIb)

in welcher

$R^{1''}$, $R^{3'}$, $R^{4'}$, $R^{5'}$, $R^{6'}$ und $R^{7'}$ die in Anspruch 9 angegebene Bedeutung haben.

12. Verfahren zur Herstellung von 5-Aminopyrazolen der Formel (IIa)

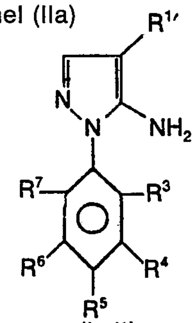

(IIa)

in welcher

$R^{1'}$ für jeweils geradkettiges oder verzweigtes Alkoxycarbonyl, Alkenyloxycarbonyl, Alkinyloxycarbonyl, Alkenylaminocarbonyl oder Alkinylaminocarbonyl mit jeweils bis zu 4 Kohlenstoffatomen in den einzelnen Alkylteilen steht, und

$R^3$, $R^4$, $R^5$, $R^6$ und $R^7$ die in Anspruch 1 und 4 angegebene Bedeutung haben, und wobei die Verbindung

5-Amino-4-methoxycarbonyl-1-(2-trifluormethyl-4-chlor-phenyl)-pyrazol ausgenommen ist,

dadurch gekennzeichnet, dass man Acrylnitril-Derivate der Formel (IVa),

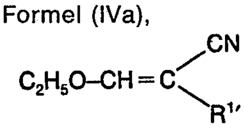

(IVa)

in welcher

$R^{1'}$ die oben angegebene Bedeutung hat,

mit Phenylhydrazinen der Formel (V),

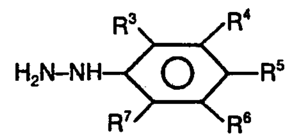

in welcher

$R^3$, $R^4$, $R^5$, $R^6$ und $R^7$ die oben angegebene Bedeutung haben,

zunächst in einer ersten Stufe, gegebenenfalls in Gegenwart eines Verdünnungsmittels, sowie gegebenenfalls in Gegenwart eines Reaktionshilfsmittels umsetzt zu den Phenylhydrazin-Derivaten der Formel (VIa)

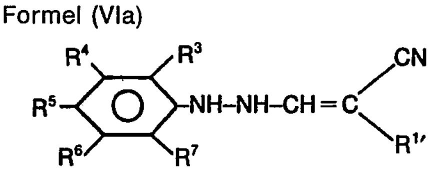

(VIa)

in welcher

$R^{1'}$, $R^3$, $R^4$, $R^5$, $R^6$ und $R^7$ die oben angegebene Bedeutung haben,

und diese in einer zweiten Stufe, gegebenenfalls

in Gegenwart eines Verdünnungsmittels cyclisiert.

13. Verfahren zur Herstellung von 5-Aminopyrazolen der Formel (IIb),

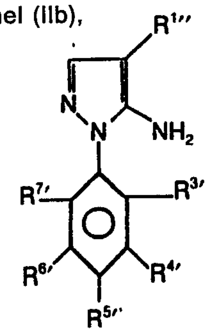

(IIb)

in welcher

$R^{1''}$ für Cyano oder für Alkylaminocarbonyl mit bis zu 4 Kohlenstoffatomen im Alkylteil steht und

$R^{3'}$, $R^{4'}$, $R^{5'}$, $R^{6'}$ und $R^{7'}$, welche gleich oder verschieden sind, für Wasserstoff, Fluor, Chlor, Brom, Jod, Cyano, Nitro, jeweils geradkettiges oder verzweigtes Alkyl, Alkoxy, Alkylsulfonyl oder Alkoxycarbonyl mit bis zu 4 Kohlenstoffatomen in den jeweiligen Alkylteilen oder für den Rest $-X-R^8$ stehen, wobei X für Sauerstoff, Schwefel, Sulfinyl oder Sulfonyl steht und $R^8$ für Halogenalkyl mit bis zu 4 Kohlenstoffatomen und bis zu 9 gleichen oder verschiedenen Halogenatomen steht mit der Massgabe, dass mindestens einer der Reste $R^{3'}$, $R^{4'}$, $R^{5'}$, $R^{6'}$ oder $R^{7'}$ für einen Rest $-X-R^8$ steht, wobei nicht gleichzeitig X für Sauerstoff und $R^8$ für Trifluormethyl stehen,

dadurch gekennzeichnet, dass man Acrylnitril-Derivate der Formel (IVb),

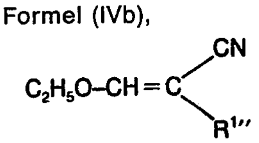

(IVb)

in welcher

$R^{1''}$ die oben angegebene Bedeutung hat,

mit Phenylhydrazinen der Formel (Va),

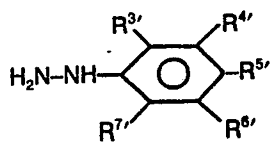

(Va)

in welcher

$R^{3'}$, $R^{4'}$, $R^{5'}$, $R^{6'}$ und $R^{7'}$ die oben angegebene Bedeutung haben,

zunächst in einer ersten Stufe, gegebenenfalls in Gegenwart eines Verdünnungsmittels und gegebenenfalls in Gegenwart eines Reaktionshilfsmittels umsetzt zu den Phenylhydrazin-Derivaten der Formel (VIb),

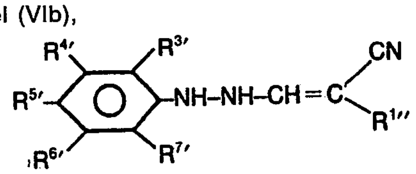

(VIb)

in welcher

$R^{1''}$, $R^{3'}$, $R^{4'}$, $R^{5'}$, $R^{6'}$ und $R^{7'}$ die oben angegebene Bedeutung haben,

und diese in einer 2. Stufe, gegebenenfalls in Gegenwart eines Verdünnungsmittels cyclisiert.

14. Verfahren zur Herstellung von Phenylhydrazinen der Formel (VIa)

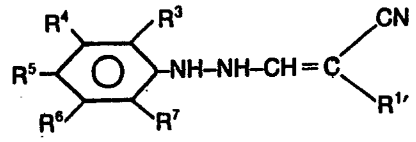

(VIa)

in welcher

$R^{1'}$, $R^3$, $R^4$, $R^5$, $R^6$ und $R^7$ die in Anspruch 4 und 12 angegebene Bedeutung haben,

dadurch gekennzeichnet, dass man Acrylnitril-Derivate der Formel (IVa),

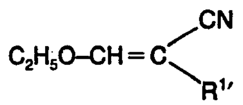

(IVa)

in welcher

$R^{1'}$ die oben angegebene Bedeutung hat,

mit Phenylhydrazinen der Formel (V),

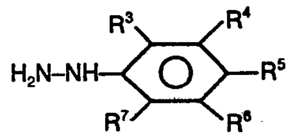

in welcher

$R^3$, $R^4$, $R^5$, $R^6$ und $R^7$ die oben angegebene Bedeutung haben,

zunächst in einer ersten Stufe, gegebenenfalls in Gegenwart eines Verdünnungsmittels, sowie gegebenenfalls in Gegenwart eines Reaktionshilfsmittels umsetzt.

15. Verfahren zur Herstellung von Phenylhydrazinen der Formel (VIb),

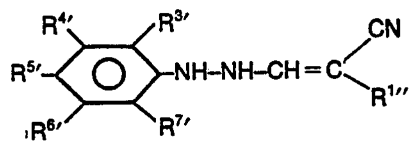

(VIb)

in welcher

$R^{1''}$, $R^{3'}$, $R^{4'}$, $R^{5'}$, $R^{6'}$ und $R^{7'}$ die in Anspruch 13 angegebene Bedeutung haben,

dadurch gekennzeichnet, dass man Acrylnitril-Derivate der Formel (IVb),

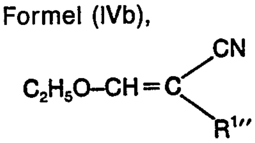

(IVb)

in welcher

$R^{1''}$ die oben angegebene Bedeutung hat,

mit Phenylhydrazinen der Formel (Va),

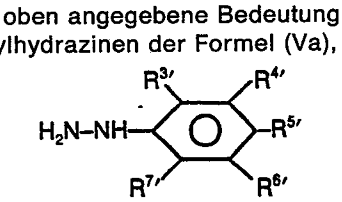

(Va)

in welcher

$R^{3'}$, $R^{4'}$, $R^{5'}$, $R^{6'}$ und $R^{7'}$ die oben angegebene Bedeutung haben,

zunächst in einer ersten Stufe, gegebenenfalls in Gegenwart eines Verdünnungsmittels und gegebenenfalls in Gegenwart eines Reaktionshilfsmittels umsetzt.

**Claims**

1. Substituted 5-acylamino-1-phenylpyrazoles of the general formula (I)

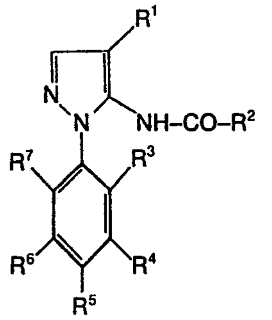

(I)

in which

$R^1$ represents cyano, aminocarbonyl or, in each case straight-chain or branched, alkoxycarbonyl, alkenyloxycarbonyl, alkinyloxycarbonyl, alkylaminocarbonyl, alkenylaminocarbonyl or alkinylaminocarbonyl having in each case up to 4 carbon atoms in the individual alkyl parts,

$R^2$ represents hydrogen, in each case straight-chain or branched alkyl, alkenyl or alkinyl having up to 6 carbon atoms, cycloalkyl having 3 to 7 carbon atoms, in each case straight-chain or branched alkoxyalkyl or alkylthioalkyl having in each case up to 4 carbon atoms in the individual alkyl parts, or straight-chain or branched halogenoalkyl having up to 6 carbon atoms and up to 9 identical or different halogen atoms, or represents phenyl which is optionally monosubstituted or polysubstituted by identical or different substituents, possible substituents being: halogen, cyano, nitro and in each case straight-chain or branched alkyl, alkoxy, alkylthio or alkoxycarbonyl having in each case up to 4 carbon atoms in the individual alkyl parts, and

$R^3$, $R^4$, $R^5$, $R^6$ and $R^7$ independently of one another represent hydrogen, fluorine, chlorine, bromine, iodine, cyano, nitro, in each case straight-chain or branched alkyl, alkoxy, alkylsulphonyl or alkoxycarbonyl having up to 4 carbon atoms in the individual alkyl parts, or a radical $-(X)_n-R^8$, wherein

X represents oxygen, sulphur, sulphinyl or sulphonyl,

n represents 0 or 1 and

$R^8$ represents straight-chain or branched halogenoalkyl having up to 4 carbon atoms and up to 9 identical or different halogen atoms, with the proviso that at least one of the radicals $R^3$, $R^4$, $R^5$, $R^6$ or $R^7$ represents a radical $-(X)_n-R^8$, and $R^1$ does not represent cyano if $R^5$ represents trifluoromethyl.

2. 5-Propionylamino-4-cyano-1-(2,6-dichloro-4-trifluoromethylthio-phenyl)-pyrazole (1) according to Claim 1,

3. 5-Propionylamino-4-cyano-1-(2,4-di-(trifluoromethyl)-phenyl-pyrazole.

4. Process for the preparation of substituted 5-acylamino-1-phenylpyrazols of the general formula (I)

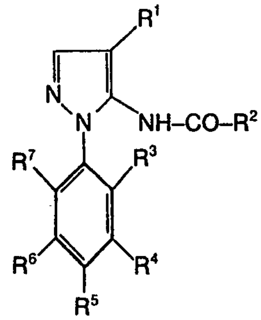

(I)

in which

$R^1$ represents cyano, aminocarbonyl or, in each case straight-chain or branched, alkoxycarbonyl, alkenyloxycarbonyl, alkinyloxycarbonyl, alkylaminocarbonyl, alkenylaminocarbonyl or alkinylaminocarbonyl having in each case up to 4 carbon atoms in the individual alkyl parts,

$R^2$ represents hydrogen, in each case straight-chain or branched alkyl, alkenyl or alkinyl having up to 6 carbon atoms, cycloalkyl having 3 to 7 carbon atoms, in each case straight-chain or branched alkoxyalkyl or alkylthioalkyl having in each case up to 4 carbon atoms in the individual alkyl parts, or straight-chain or branched halogenoalkyl having up to 6 carbon atoms and up to 9 identical or different halogen atoms, or represents phenyl which is optionally monosubstituted or polysubstituted by identical or different substituents, possible substituents being: halogen, cyano, nitro and in each case straight-chain or branched alkyl, alkoxy, alkylthio or alkoxycarbonyl having in each case up to 4 carbon atoms in the individual alkyl parts, and

$R^3$, $R^4$, $R^5$, $R^6$ and $R^7$ independently of one another represent hydrogen, fluorine, chlorine, bromine, iodine, cyano, nitro, in each case straight-chain or branched alkyl, alkoxy, alkylsulphonyl or alkoxycarbonyl having up to 4 carbon atoms in the individual alkyl parts, or a radical $-(X)_n-R^8$, wherein

X represents oxygen, sulphur, sulphinyl or sulphonyl,

n represents 0 or 1 and

$R^8$ represents straight-chain or branched halogenoalkyl having up to 4 carbon atoms and up to 9 identical or different halogen atoms, with the provisio that at least one of the radicals $R^3$, $R^4$, $R^5$, $R^6$ or $R^7$ represents a radical $-(X)_n-R^8$, and $R^1$ does not represent cyano if $R^5$ represents trifluoromethyl,

characterized in that 5-amino-pyrazoles of the formula (II)

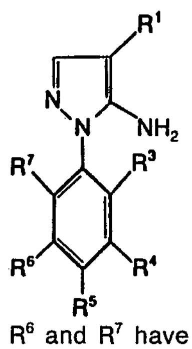

(II)

in which

$R^1$, $R^3$, $R^4$, $R^5$, $R^6$ and $R^7$ have the abovementioned meaning,

are reacted with acylating agents of the formula (III)

$R^2$–CO–A (III)

in which

$R^2$ has the abovementioned meaning and

A represents an activating leaving group, if appropriate in the presence of a diluent and if appropriate in the presence of an acid-binding agent.

5. Herbicidal agents, characterized in that they contain at least one substituted 5-acylamino-1-phenylpyrazole of the formula (I) according to Claim 1 and 4 and the compound according to Claim 3.

6. Use of substituted 5-acylamino-1-phenylpyrazoles of the formlua (I) according to Claim 1 and 4 and the compound according to Claim 3 for combating undesired plant growth.

7. Process for the preparation of herbicidal agents, characterized in that substituted 5-acylamino-1-phenylpyrazoles of the formula (I) according to Claim 1 and 4 and the compound according to Claim 3 are mixed with extenders and/or surface-active agents.

8. 5-Aminopyrazoles of the formula (IIa)

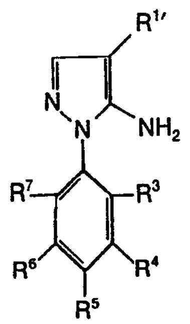

(IIa)

in which

$R^{1'}$ represents in each case straight-chain or branched alkoxycarbonyl, alkenoxycarbonyl. alkinyloxycarbonyl, alkenylaminocarbonyl or alkinylaminocarbonyl having in each case up to 4 carbon atoms in each individual alkyl parts, and

$R^3$, $R^4$, $R^5$, $R^6$ and $R^7$ have the meaning specified in Claim 1 and

wherein the compound

5-amino-4-methoxycarbonyl-1-(2-trifluoromethyl-4-chloro-phenyl)-pyrazole is excepted.

9. 5-Aminopyrazoles of the formula (IIb)

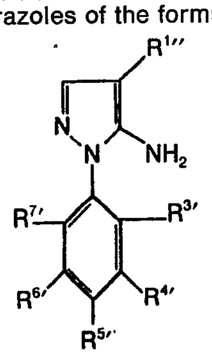

(IIb)

in which

$R^{1''}$ represents cyano or alkylaminocarbonyl having up to 4 carbon atoms in the alkyl part, and

$R^{3'}$, $R^{4'}$, $R^{5'}$, $R^{6'}$ and $R^{7'}$, which are identical or different, represent hydrogen, fluorine, chlorine, bromine, iodine, cyano, nitro, in each case straight-chain or branched alkyl, alkoxy, alkylsulphonyl or alkoxycarbonyl having up to 4 carbon atoms in the individual alkyl parts, or the radical $-X-R^8$,

wherein

X represents oxygen, sulphur, sulphinyl or sulphonyl and

$R^8$ represents halogenalkyl having up to 4 carbon atoms and up to 9 identical or different halogen atoms,

with the proviso that at least one of the radicals $R^{3'}$, $R^{4'}$, $R^{5'}$, $R^{6'}$ or $R^{7'}$ represents a radical $-X-R^8$, and X does not represent oxygen if $R^8$ represents trifluoromethyl.

10. Phenylhydrazine derivatives of the formula (VIa)

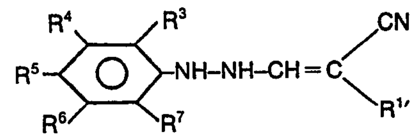

(VIa)

in which

$R^{1'}$, $R^3$, $R^4$, $R^5$, $R^6$ and $R^7$ have the meaning specified in Claim 8 and 1, respectively.

11. Phenylhydrazine derivatives of the formula (VIb)

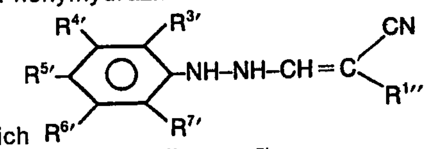

(VIb)

in which

$R^{1''}$, $R^{3'}$, $R^{4'}$, $R^{5'}$, $R^{6'}$ and $R^{7'}$, have the meaning specified meaning specified in Claim 9.

12. Process for the preparation of 5-aminopyrazoles of the formula (IIa)

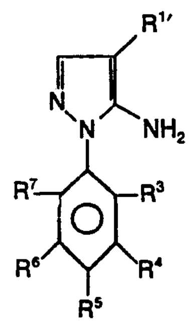

(IIa)

in which

$R^{1'}$ represents in each case straight-chain or branched alkoxycarbonyl, alkenyloxycarbonyl, alkinyloxycarbonyl, alkenylaminocarbonyl or alkinylaminocarbonyl having in each case up to 4 carbon atoms in the individual alkyl parts, and

$R^3$, $R^4$, $R^5$, $R^6$ and $R^7$ have the meaning specified in Claim 1 and 4 and wherein the compound

5-amino-4-methoxycarbonyl-1-(2-trifluoromethyl-4-chloro-phenyl)-pyrazole

is excepted, characterized in that acrylonitrile derivatives of the formula (IVa)

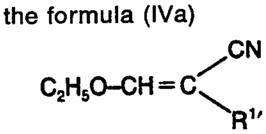

(IVa)

in which

$R^{1'}$ has the abovementioned meaning,

are reacted with phenylhydrazines of the formula (V),

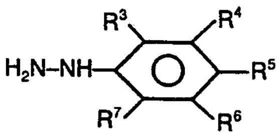

in which

$R^3$, $R^4$, $R^5$, $R^6$ and $R^7$ have the abovementioned meaning,

initially in a first stage, if appropriate in the presence of a diluent, and if appropriate in the presence of a reaction auxiliary, to give the phenylhydrazine derivatives of the formula (VIa)

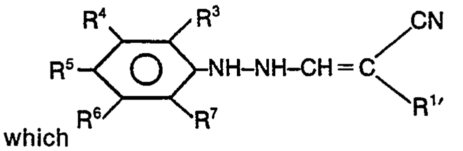 (VIa)

in which

$R^{1'}$, $R^3$, $R^4$, $R^5$, $R^6$ and $R^7$ have the abovementioned meaning,

and these are cyclized in a second stage, if appropriate in the presence of a diluent.

13. Process for the preparation of 5-aminopyrazoles of the formula (IIb)

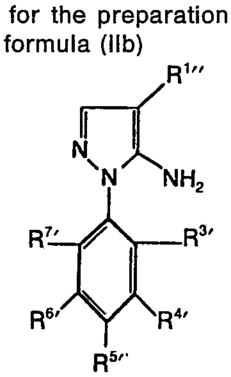 (IIb)

in which

$R^{1''}$ represents cyano or alkylaminocarbonyl having up to 4 carbon atoms in the alkyl part, and

$R^{3'}$, $R^{4'}$, $R^{5'}$, $R^{6'}$ and $R^{7'}$, which are identical or different, represent hydrogen, fluorine, chlorine, bromine, iodine, cyano, nitro, in each case straight-chain or branched alkyl, alkoxy, alkylsulphonyl or alkoxycarbonyl having up to 4 carbon atoms in the individual alkyl parts, or the radical $-X-R^8$,

wherein

X represents oxygen, sulphur, sulphinyl or sulphonyl and

$R^8$ represents halogenalkyl having up to 4 carbon atoms and up to 9 identical or different halogen atoms, with the proviso that at least one of the radicals $R^{3'}$, $R^{4'}$, $R^{5'}$, $R^{6'}$ or $R^{7'}$ represents a radical $-X-R^8$, and X does not represent oxygen if $R^8$ represents trifluoromethyl,

characterized in that acrylonitrile derivatives of the formula (IVb)

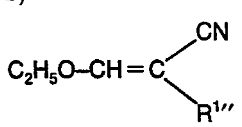 (IVb)

in which

$R^{1''}$ has the abovementioned meaning,

are reacted with phenylhydrazines of the formula (Va)

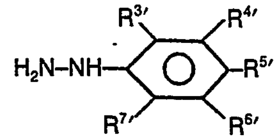 (Va)

in which

$R^{3'}$, $R^{4'}$, $R^{5'}$, $R^{6'}$ and $R^{7'}$ have the abovementioned meaning,

initially in a first stage, if appropriate in the presence of a diluent and if appropriate in the presence of a reaction auxiliary, to give the phenylhydrazine derivatives of the formula (VIb)

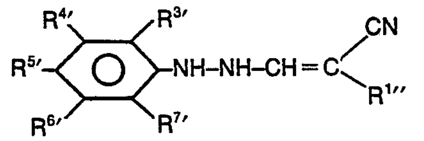 (VIb)

in which

$R^{1''}$, $R^{3'}$, $R^{4'}$, $R^{5'}$, $R^{6'}$ and $R^{7'}$ have the abovementioned meaning,

and these are cyclized in a second stage, if appropriate in the presence of a diluent.

14. Process for the preparation of phenylhydrazines of the formula (VIa)

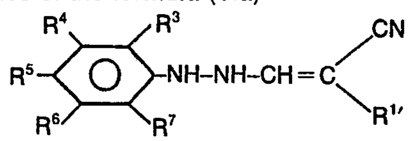 (VIa)

in which

$R^{1'}$, $R^3$, $R^4$, $R^5$, $R^6$ and $R^7$ have the meaning specified in Claim 4 and 12,

characterized in that acrylonitrile derivatives of the formula (IVa)

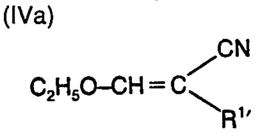 (IVa)

in which

$R^{1'}$ has the abovementioned meaning,

are reacted with phenylhydrazines of the formula (V)

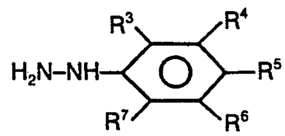

in which

$R^3$, $R^4$, $R^5$, $R^6$ and $R^7$ have the abovementioned meaning,

initially in a first stage, if appropriate in the presence of a diluent and if appropriate in the presence of a reaction auxiliary.

15. Process for the preparation of phenylhydrazines of the formula (VIb)

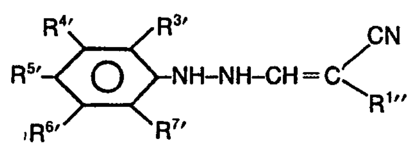 (VIb)

in which

$R^{1''}$, $R^{3'}$, $R^{4'}$, $R^{5'}$, $R^{6'}$ and $R^{7'}$ have the meaning specified in Claim 13,

characterized in that acrylonitrile derivatives of the formula (IVb)

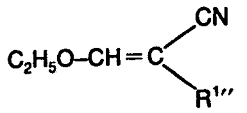

(IVb)

in which

$R^{1'''}$ has the abovementioned meaning,

are reacted with phenylhydrazines of the formula (Va)

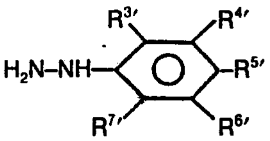

(Va)

in which

$R^{3'}$, $R^{4'}$, $R^{5'}$, $R^{6'}$ and $R^{7'}$ have the abovementioned meaning,

initially in a first stage, if appropriate in the presence of a diluent and if appropriate in the presence of a reaction auxiliary.

**Revendications**

1. 5-acylamino-1-phénylpyrazoles substitués de formule générale (I)

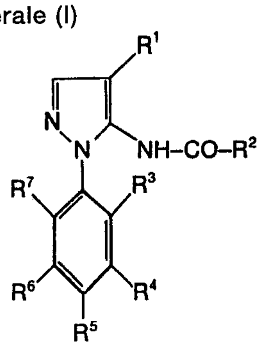

(I)

dans laquelle

$R^1$ représente un groupe cyano, un groupe aminocarbonyle ou un groupe alcoxycarbonyle, alcényloxycarbonyle, alcynyloxycarbonyle, alkylaminocarbonyle, alcénylaminocarbonyle ou alcynylaminocarbonyle, chacun linéaire ou ramifié et comportant chacun jusqu'à 4 atomes de carbone dans les divers fragments alkyles,

$R^2$ représente un atome d'hydrogène, un groupe alkyle, alcényle, ou alcynyle, chaque fois linéaire ou ramifié et comportant jusqu'à 6 atomes de carbone, un groupe cycloalkyle ayant 3 à 7 atomes de carbone, un groupe alcoxyalkyle ou alkylthioalkyle, chacun linéaire ou ramifié et comportant chacun jusqu'à 4 atomes de carbone dans les divers fragments alkyles, ou un groupe halogénoalkyle, linéaire ou ramifié, comportant jusqu'à 6 atomes de carbone et jusqu'à 9 atomes d'halogène identiques ou différents, ou bien un groupe phényle portant un ou plusieurs substituants identiques ou différents, et l'on peut citer comme substituant: un halogène, un groupe cyano, nitro, un groupe alkyle, alcoxy, alkylthio ou alcoxycarbonyle, chacun linéaire ou ramifié et ayant jusqu'à 4 atomes de carbone dans les divers fragments alkyles et

$R^3$, $R^4$, $R^5$, $R^6$ et $R^7$ représentent, indépendamment l'un de l'autre, un atome d'hydrogène, de fluor, de chlore, de brome, ou d'iode, un groupe cyano, nitro, un groupe alkyle,, alcoxy, alkylsulfonyle ou alcoxycarbonyle, chacun linéaire ou ramifié et ayant jusqu'à 4 atomes de carbone dans les divers fragments alkyles, ou bien un reste $-(X)_n-R^8$, dans lequel

X représente un atome d'oxygène ou de soufre ou un groupe sulfinyle ou sulfonyle,

n vaut 0 ou 1 et

$R^8$ représente un groupe halogénoalkyle, linéaire ou ramifié, comportant jusqu'à 4 atomes de carbone, et jusqu'à 9 atomes d'halogènes, identiques ou différents, à la condition qu'au moins l'un des restes $R^3$, $R^4$, $R^5$, $R^6$ ou $R^7$ représente un reste $-(X)_n-R^8$, mais cependant, $R^1$ ne devant pas représenter un groupe cyano en même temps que $R^5$ représenterait un groupe trifluorométhyle.

2. Le 5-propionylamino-4-cyano-1-(2,6-dichloro-4-trifluorométhylthio-phényl)-pyrazole (1) selon la revendication 1.

3. Le 5-propionylamino-4-cyano-(2,4-di-(trifluorométhyl)-phényl-pyrazole.

4. Procédé pour préparer des 5-acylamino-1-phénylpyrazoles substitués, de formule générale (I),

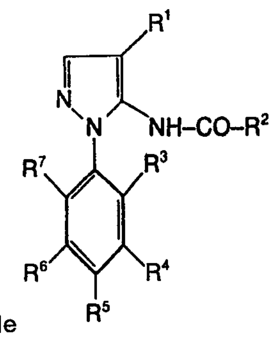

(I)

dans laquelle

$R^1$ représente un groupe cyano, un groupe aminocarbonyle ou un groupe alcoxycarbonyle, alcényloxycarbonyle, alcynyloxycarbonyle, alkylaminocarbonyle, alcénylaminocarbonyle, ou alcynylaminocarbonyle, chacun linéaire ou ramifié et comportant chacun jusqu'à 4 atomes de carbone dans les divers fragments alkyles,

$R^2$ représente un atome d'hydrogène, un groupe alkyle, alcényle ou alcynyle, linéaire ou ramifié et ayant chacun jusqu'à 6 atomes de carbone, un groupe cycloalkyle comportant 3 à 7 atomes de carbone, un groupe alcoxyalkyle ou alkylthioalkyle linéaire ou ramifié et comportant chacun jusqu'à 4 atomes de carbone dans les divers fragments alkyles ou un groupe halogénoalkyle, linéaire ou ramifié, comportant jusqu'à 6 atomes de carbone et jusqu'à 9 atomes d'halogènes, identiques ou différents, ou un groupe phényle, comportant un ou plusieurs substituants identiques ou différents, et l'on peut citer alors comme substituants: un atome d'halogène, un groupe cyano, nitro, un groupe alkyle, alcoxy, alkylthio ou alcoxycarbonyle, chacun linéaire ou ramifié et comportant chacun jusqu'à 4 atomes de carbone dans les divers fragments alkyles et

$R^3$, $R^4$, $R^5$, $R^6$ et $R^7$ représentent, indépendamment l'un de l'autre, un atome d'hydrogène, de fluor, de chlore, de brome, d'iode, un groupe cyano, un groupe nitro, un groupe alkyle, alcoxy, alkylsulfonyle ou alcoxycarbonyle, linéaires ou ramifiés et comportant chacun jusqu'à 4 atomes

de carbone dans les divers fragments alkyles, ou un reste $-(X)_n-R^8$, dans lequel,

X représente un atome d'oxygène ou de soufre ou un groupe sulfinyle ou sulfonyle,

n vaut 0 ou 1 et

$R^8$ représente un groupe halogéno-alkyle linéaire ou ramifié ayant jusqu'à 4 atomes de carbone et jusqu'à 9 atomes d'halogène, identique ou différents, à la condition qu'au moins l'un des restes $R^3$, $R^4$, $R^5$, $R^6$ ou $R^7$ représente un reste $-(X)_n-R^8$, mais cependant $R^1$ ne pouvant simultanément représenter un groupe cyano alors que $R^5$ représenterait un groupe trifluorométhyle,
procédé caractérisé en ce qu'on fait réagir, éventuellement en présence d'un diluant, et éventuellement en présence d'un agent de fixation des acides, des 5-amino-pyrazoles de formule (II),

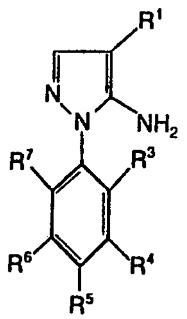

(II)

dans laquelle

$R^1$, $R^3$, $R^4$, $R^5$, $R^6$ et $R^7$ ont le sens indiqué ci-dessus,
avec des agents d'acylation de formule (III),

$$R^2\text{-CO-A} \qquad (III)$$

dans laquelle

$R^2$ a le sens indiqué ci-dessus, et

A représente un groupe partant, à rôle d'activation.

5. Produit herbicide, caractérisé par une teneur en au moins un 5-acylamino-1-phénylpyrazole substitué, de formule (I) selon les revendications 1 et 4 et le composé selon la revendication 3.

6. Utilisation de 5-acylamino-1-phénylpyrazoles substitués, de formule (I), selon les revendications 1 et 4, et du composé selon la revendication 3, pour combattre la croissance de plantes non souhaitées.

7. Procédé pour préparer des produits herbicides, caractérisé en ce qu'on mélange des 5-acylamino-1-phénylpyrazoles substitués de formule (I) selon les revendications 1 et 4, et le composé selon la revendication 3, avec des agents d'allongement et/ou des agents tensio-actifs.

8. 5-aminopyrazoles de formule (IIa)

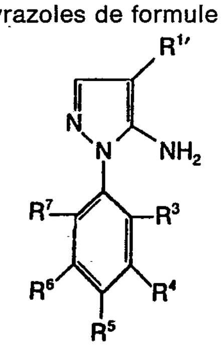

(IIa)

dans laquelle

$R^1$ représente un groupe alcoxycarbonyle, alcényloxycarbonyle, alcynyloxycarbonyle, alcénylaminocarbonyle ou alcynylaminocarbonyle, chacun linéaire ou ramifié et comportant à chaque fois jusqu'à 4 atomes de carbone dans les divers fragments alkyles et,

$R^3$, $R^4$, $R^5$, $R^6$ et $R^7$ ayant le sens indiqué à la revendication 1, et à l'exclusion du composé 5-amino-4-méthoxycarbonyl-1-(2-trifluorométhyl-4-chloro-phényl)-pyrazole.

9. 5-aminopyrazoles de formule (IIb)

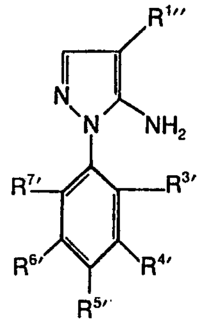

(IIb)

dans laquelle

$R^{1''}$ représente un groupe cyano ou un groupe alkylaminocarbonyle ayant jusqu'à 4 atomes de carbone dans le fragment alkyle, et

$R^{3'}$ $R^{4'}$, $R^{5'}$, $R^{6'}$ et $R^{7'}$, qui sont identiques ou différents, représentent chacun un atome d'hydrogène, de fluor, de chlore, de brome ou d'iode ou un groupe cyano, nitro, un groupe alkyle, alcoxy, alkylsulfonyle ou alcoxycarbonyle, chaque fois linéaire ou ramifié et ayant jusqu'à 4 atomes de carbone dans les divers fragments alkyles, ou bien le reste $-X-R^8$, dans lequel X représente un atome d'oxygène ou de soufre ou un groupe sulfinyle ou sulfonyle et $R^8$ représente un groupe halogéno-alkyle ayant jusqu'à 4 atomes de carbone et jusqu'à 9 atomes d'halogènes, identiques ou différents, à la condition qu'au moins l'un des restes $R^{3'}$, $R^{4'}$, $R^{5'}$, $R^{6'}$ ou $R^{7'}$ représente un reste $-X-R^8$, mais, simultanément, X ne pouvant représenter un atome d'oxygène lorsque $R^8$ représente un groupe trifluorométhyle.

10. Dérivés de la phénylhydrazine, de formule (VIa)

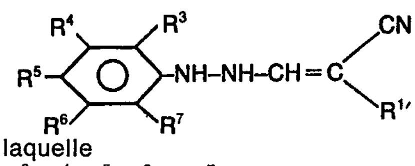

(VIa)

dans laquelle

$R^1$, $R^3$, $R^4$, $R^5$, $R^6$ et $R^7$ ont le sens indiqué à la revendication 8 ou à la revendication 1.

11. Dérivés de la phénylhydrazine de formule (VIb)

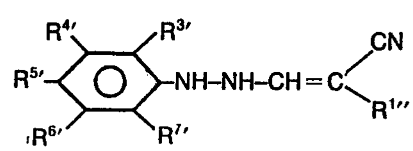

(VIb)

dans laquelle

$R^{1''}$, $R^{3'}$, $R^{4'}$, $R^{5'}$, $R^{6'}$ et $R^{7'}$ ont le sens indiqué à la revendication 9.

12. Procédé pour préparer des 5-aminopyrazoles de formule (IIa)

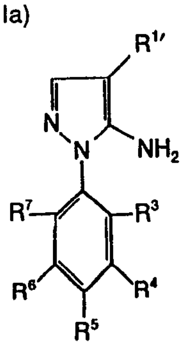

(IIa)

dans laquelle

$R^{1'}$ représente un groupe alcoxycarbonyle, alcényloxycarbonyle, alcynyloxycarbonyle, alcénylaminocarbonyle, ou alcynylaminocarbonyle, chacun linéaire ou ramifié et comportant à chaque fois jusqu'à 4 atomes de carbone dans les divers fragments alkyles, et

$R^3$, $R^4$, $R^5$, $R^6$ et $R^7$ ont le sens indiqué à la revendication 1 et à la revendication 4, et à l'exclusion du composé

5-amino-4-méthoxycarbonyl-1-(2-trifluorométhyl-4-chlorophényl)-pyrazole,

procédé caractérisé en ce qu'on fait réagir des dérivés de l'acrylonitrile de formule (IVa),

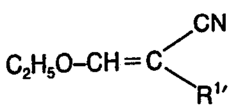

(IVa)

dans laquelle

$R^{1'}$ a le sens indiqué ci-dessus, avec des phénylhydrazines de formule (V)

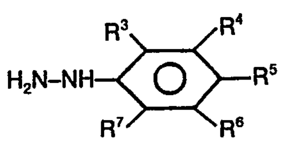

dans laquelle

$R^3$, $R^4$, $R^5$, $R^6$ et $R^7$ ont le sens indiqué ci-dessus, tout d'abord dans une première étape en opérant éventuellement en présence d'un diluant et éventuellement en présence d'un adjuvant favorisant la réaction, pour obtenir les dérivés de phénylhydrazine de formule (VIa)

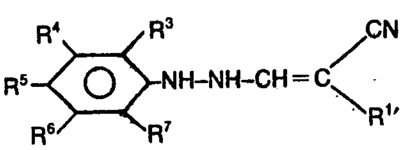

(VIa)

dans laquelle

$R^{1'}$, $R^3$, $R^4$, $R^5$, $R^6$ et $R^7$ ont le sens indiqué ci-dessus,

et, dans une seconde étape, on cyclise ce composé, en opérant éventuellement en présence d'un diluant.

13. Procédé pour préparer des 5-aminopyrazoles de formule (IIb)

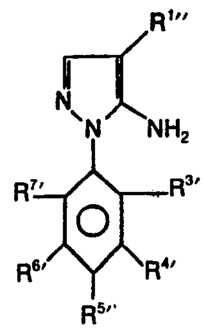

(IIb)

dans laquelle

$R^{1''}$ représente un groupe cyano ou un groupe alkylaminocarbonyle comportant jusqu'à 4 atomes de carbone dans le fragment alkyle et

$R^{3'}$, $R^{4'}$, $R^{5'}$, $R^{6'}$ et $R^{7'}$, qui sont identiques ou différents, représentent chacun un atome d'hydrogène, de fluor, de chlore, de brome ou d'iode ou un groupe cyano, nitro, un groupe alkyle, alcoxy, alkylsulfonyle, ou alcoxycarbonyle, chacun linéaire ou ramifié et comportant jusqu'à 4 atomes de carbone dans les divers fragments alkyles, ou bien le reste $-X-R^8$, où X représente un atome d'oxygène ou de soufre ou un groupe sulfinyle ou sulfonyle et $R^8$ représente un groupe halogénoalkyle ayant jusqu'à 4 atomes de carbone et jusqu'à 9 atomes d'halogènes, identiques ou différents, à la condition qu'au moins l'un des restes $R^{3'}$, $R^{4'}$, $R^{5'}$, $R^{6'}$ ou $R^{7'}$ représentent un reste $-X-R^8$, et X ne pouvant pas représenter un atome d'oxygène en même temps que $R^8$ représenterait un groupe trifluorométhyle;

procédé caractérisé en ce qu'on fait réagir des dérivés de l'acrylonitrile de formule (IVb)

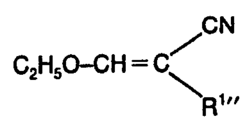

(IVb)

dans laquelle

$R^{1''}$ a le sens indiqué ci-dessus,

avec des phénylhydrazines de formule (Va)

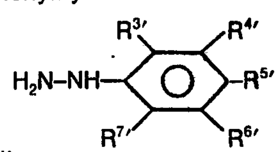

(Va)

dans laquelle

$R^{3'}$, $R^{4'}$, $R^{5'}$, $R^{6'}$ et $R^{7'}$ ont le sens indiqué ci-dessus,

tout d'abord, en opérant dans une première étape, éventuellement en présence d'un diluant et éventuellement en présence d'un adjuvant favorisant la réaction, pour obtenir les dérivés de la phénylhydrazine de formule (VIb)

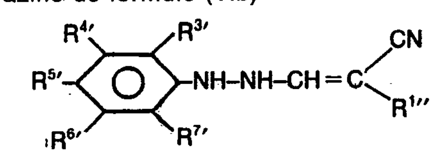

(VIb)

dans laquelle

$R^{1''}$, $R^{3'}$, $R^{4'}$, $R^{5'}$, $R^{6'}$ et $R^{7'}$, ont le sens indiqué ci-dessus, et l'on cyclise ce composé, dans une seconde étape, éventuellement en présence d'un diluant.

14. Procédé pour préparer des phénylhydrazines de formule (VIa)

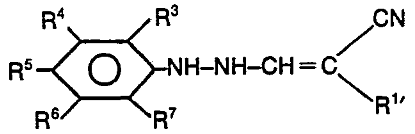

(VIa)

dans laquelle

$R^{1'}$, $R^3$, $R^4$, $R^5$, $R^6$ et $R^7$ ont le sens indiqué aux revendications 4 et 12,

procédé caractérisé en ce qu'on fait réagir des dérivés de l'acrylonitrile, de formule (IVa)

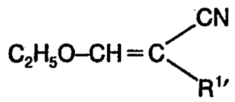

(IVa)

dans laquelle

$R^{1'}$ a le sens indiqué ci-dessus,

avec des phénylhydrazines de formule (V)

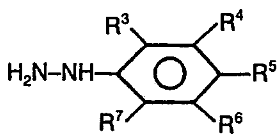

dans laquelle

$R^3$, $R^4$, $R^5$, $R^6$ et $R^7$ ont le sens indiqué ci-dessus, tout d'abord dans une première étape en opérant éventuellement en présence d'un diluant, ainsi qu'éventuellement en présence d'un adjuvant de réaction.

15. Procédé pour préparer des phénylhydrazines de formule (VIb)

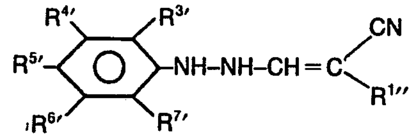

(VIb)

dans laquelle

$R^{1''}$, $R^{3'}$, $R^{4'}$, $R^{5'}$, $R^{6'}$ et $R^{7'}$ ont le sens indiqué à la revendication 13,

procédé caractérisé en ce qu'on fait réagir des dérivés de l'acrylonitrile de formule (IVb)

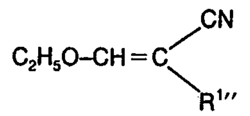

(IVb)

dans laquelle

$R^{1''}$ a le sens indiqué ci-dessus,

avec des phénylhydrazines de formule (Va)

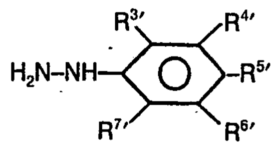

(Va)

dans laquelle

$R^{3'}$, $R^{4'}$, $R^{5'}$, $R^{6'}$ et $R^{7'}$ ont le sens indiqué ci-dessus,

en opérant tout d'abord dans une première étape éventuellement en présence d'un diluant et éventuellement en présence d'un adjuvant favorisant la réaction.